Europäisches Patentamt

European Patent Office

Office européen des brevets

(19)

(11) Publication number: **0 361 960**
**A2**

(12) **EUROPEAN PATENT APPLICATION**

(21) Application number: **89309983.8**

(51) Int. Cl.⁵: **G01R 33/48 , A61K 49/00**

(22) Date of filing: **29.09.89**

(30) Priority: **29.09.88 US 252565**

(43) Date of publication of application:
**04.04.90 Bulletin 90/14**

(84) Designated Contracting States:
**AT BE CH DE ES FR GB GR IT LI LU NL SE**

(71) Applicant: **RANNEY, David F.**
**3539 Courtdale Drive**
**Dallas, TX 75234(US)**

(72) Inventor: **RANNEY, David F.**
**3539 Courtdale Drive**
**Dallas, TX 75234(US)**

(74) Representative: **Beresford, Keith Denis Lewis et al**
**BERESFORD & Co. 2-5 Warwick Court High Holborn**
**London WC1R 5DJ(GB)**

(54) **Methods and compositions for magnetic resonance imaging.**

(57) Improved compositions and methods for selective access to tumor regions (or other regions of abnormal endothelial properties). This capability provides powerful contrast-enhancement agents for nuclear magnetic resonance imaging. A polyatomic complex which includes intramolecular ferromagnetic coupling between metal atoms is associated with a polymer or microsphere carrier matrix which will bind to endothelial determinants. A solution containing this carrier complex is injected into a human (or other) body to be imaged. The carrier complex will preferentially extravasate at locations where the blood vessel walls have increased porosity or microvascular surface changes, and especially at tumor sites. Thus, the changes in relaxation time induced by the presence of the carrier complex will provide a high-gain marker for magnetic resonance imaging.

Multiple superparamagnetic polyatomic complexes are described, including novel complexes which include acetate and glycinate bridging ligands with a polyatomic metal-atom-complex core.

EP 0 361 960 A2

## METHODS AND COMPOSITIONS FOR MAGNETIC RESONANCE IMAGING

### BACKGROUND AND SUMMARY OF THE INVENTION

The present invention relates to nuclear magnetic resonance imaging methods. .

### Nuclear Magnetic Resonance Imaging Generally

Atoms which have a magnetic moment will have sharply defined frequencies of nuclear oscillation in a strong magnetic field. This phenomenon is known as "nuclear magnetic resonance," or NMR. The frequency of oscillation of each atomic nucleus will depend on its mass, its dipole moment, the chemical bonding of the atom, the atom's environment (which will be affected by electromagnetic coupling to other atoms in the vicinity), and the strength of the magnetic field seen by the atom. Thus, the frequency of oscillation will be characteristic, not only of the various atomic species, but also of their molecular environments. By resonantly exciting these oscillations, the atomic species and their environments can be determined with accuracy.

If a pulse of RF energy is applied at a resonance frequency of atoms of a particular species and environment (e.g. hydrogen atoms in a water environment), the atomic nuclei of this type and environment will be excited into nuclear oscillation, and will later make a transition back to a low state of excitation. This transition will often be accompanied by emission of a radio-frequency signal, at the excitation frequency or a known lower frequency. (This emission is known as a "spin echo.") The spatial distribution of these "echoes" will provide a map of the distribution of atoms of the predetermined type and environment. Moreover, the time delay before the spin echo emission occurs will also give important information about the environment of the atom. (This time delay is referred to as the relaxation time.) Relaxation time analysis is generally used to provide additional detail in NMR imaging for medical purposes.

In the last decade, this technique has been applied to perform medical imaging. Living bodies can be imaged without harm, using the known resonance characteristics of (usually) protons in an aqueous environment. A strong DC magnetic field $B_0$ is used, together with gradient fields which are controlled so that the net total magnetic field will reach a predetermined value only in desired locations. A series of RF pulses is applied, while the magnetic bias fields are varied (using field-gradient coils) and the "spin echos" are detected. Since the magnetic field contours, and the resonance characteristics of the protons, are accurately known, the position where the spin echos originate can be accurately determined. By repeated pulse and measurement operations, a map of the distribution of aqueous protons (and, in some cases, of lipid protons) can be obtained. This imaging technique is referred to as "magnetic resonance imaging," or "MRI."

Much recent recent work has developed refined techniques, based on the general technique just described. The NMR phenomenon can be used to form images based merely on intensity, or time-domain windowing can be used to distinguish among atoms with differing relaxation times. Another variation is to precisely distinguish the spectral characteristics which are determined by the atomic environment of the nucleus, and this technique is referred to as spectral-shift imaging.

Magnetic resonance intensity, relaxation and spectral-shift images have been shown in recent years to provide an important, safe mode of brain and body imaging at very high spatial resolution (typically less than 0.6 x 0.6 mm in-plane resolution x 2 mm-thick slices) of internal structures, organs and pathologic foci in live animals and humans. MRI potentially allows much smaller structures and tumors to be detected and monitored therapeutically than is possible by computed axial tomography (CAT) or radionuclide imaging, which typically resolve tumor masses only at 1 to 2.5 $cm^3$.

Clinical MRI is a rapidly growing form of brain and body imaging. This has proven to be a very useful diagnostic technique, and its use is rapidly increasing. The use of this technique for body imaging is beginning to increase above 10% of total cases. This increase has been accelerated as a result of the availability, on many of the standard clinical instruments, of such improved techniques as: more rapid, T1-weighted, narrow-flip-angle pulse sequences; efficient data processing; and respiratory and cardiac gating devices. Rapid images of acceptable quality can be acquired using near-real-time acquisitions of 15 seconds (a single breathholding interval) to 2 minutes. Proton MRI detects chemical parameters in the immediate environment around mobile protons of body water (principally) and fat. Changes in these parameters are often more sensitive, and frequently occur earlier in the course of disease, than those detected by CAT (tissue densities) or radionuclide imaging (gamma isotopic emissions from radionuclides localized in diseased tissues at sufficient concentrations above blood background). (See Runge et al., 141 Am. J. Radiol. 1209 (1983)). Nevertheless, in the absence of contrast agents, it has been difficult for MRI to detect very small tumors, especially those less than 2 $mm^3$ in brain and less than 5 $mm^3$ in the body.

Body MR images are less well resolved than brain images, for several reasons. Body imaging involves larger RF coils, with resulting lower magnetic field homogeneity and decreased sensitivity (decreased signal-to-noise ratio). Body imaging also can be affected by body motion artifacts. (The time required to do NMR body imagining on human patients is typically in the range of 1.5 to 7 minutes, depending on the imaging sequence used.) Additionally, it has been demonstrated that it is difficult for MRI (unless augmented by non-electronic means) to: 1) distinguish between viable (perfused) and necrotic (unperfused) tumor; 2) recognize biologically relevant tumor subregions which must be monitored at the very small sizes (1 $mm^3$) and early intervals (20 to 30 hours) required to assess early treatment effects before tumor nodules regrow to X-ray-detectable and radionuclide-detectable sizes. The newly expanded repertoire of RF pulse sequences cannot compensate entirely for body motion artifacts. ·

## NMR Image Enhancement

It has long been suggested that strongly paramagnetic species could be used as image-enhancers for nuclear magnetic resonance (NMR) imaging of living organisms. For example, efforts have been made to transport gadolinium ions (as ion chelates) into imaging sites, since gadolinium ions are strongly paramagnetic. However, it has not heretofore been possible to achieve highly selective transport of appropriate gadolinium-bearing species to the desired imaging sites.

Image contrast enhancement with potent, nontoxic, tumor-selective MRI contrast agents can overcome many of these problems by enhancing critical tumor structures (including tumor margins) and thereby shortening image-acquisition times.

Paramagnetic contrast agents decrease the relaxation time (T1-time preferentially) of mobile, rf-pulsed protons. This increases the local image intensity of tissues, organs, tumor matrix or tumor cells in which the agent becomes localized. The result is that small tumors (and other pathologic foci) are imaged (or their spectra monitored) with improved selectivity, sensitivity and precision of marginal definition.

Seven factors make it highly desirable to develop nontoxic MRI image-enhancing agents analogous to those available for CAT.

1. They increase the specificity of MRI diagnosis.

2. Smaller lesions can be identified earlier.

3. Image-enhancing agents enhance tumor masses differently than surrounding edema fluid or abscesses. This allows the extent and invasion of tumors to be defined more precisely. Lesions with infiltrative-type growth (e.g., certain metastic carcinomas and glioblastomas) will require contrast agents for demarcation between tumor and edema fluid (Felix et al., 2 Proc. Soc. Mag. Res. Med. 831 (1985)).

4. Image-enhancing agents improve the distinction between recurrent tumor and fibrous tissue resulting from surgery and radiation.

5. Image-enhancing agents can decrease the time required per scan and potentially decrease the number of scans required per procedure. This increases the volume of procedures and decreases their expense.

6. Body imaging has a significantly lower resolution (typically 0.5-1.0 cm) and sensitivity (decreased signal-to-noise ratio) than brain imaging (see Wesbey et al., 149 Radiology 175 (1983), which is hereby incorporated by reference)). These differences result from the greater inhomogeneity of the magnetic field; the larger radio frequency coil; unequal phase-pulsing of deep versus shallow nuclei; and motion artifacts produced by respiration, cardiac systole, gastrointestinal peristalsis, and voluntary muscle movement.

7. Advanced (polymeric and microsphere) forms of contrast agents (see below) appear to be required for the optimal acquisition and interpretation of blood-flow and tissue-perfusion images and related spectral (phase) information.

Toxicity of the image-enhancing substance is not only undesirable per se, but also will necessarily limit the maximum dose which can be used, and therefore will limit the degree of image-enhancement which can be achieved.

The discrete intensities of a two-dimensional, Fourier-transformed image are described by the following general equation (for spin-echo pulse sequences):

Intensity = N(H)•f(v)•exp(-TE/T2)•(1 - exp(TE-TR/T1),

3

where:

N(H) = number of protons in the discrete tissue volume (spin density);

f(v) = a function of proton velocity and the fraction of protons which are moving (e.g., due to the following blood);

TE = time between the radio frequency (rf) pulse and the detection of signal (spin-echo);

TR = the interval between repetition of the RF pulse.

T1 = the time interval associated with the rate of proton energy transfer to the surrounding chemical environment (spin-lattice relaxation);

T2 = the time interval associated with the rate of proton energy level transfer (spin-spin relaxation).

The T1 and T2 times have reciprocal effects on image intensity. Intensity is increased by either shortening the T1 time or lengthening the T2 time. Tissue contrast occurs naturally and is related to variations in the chemical environments around water protons (major contributor) and lipid protons (usually minor). Chemical agents have been used to enhance this natural contrast. The one most widely tested clinically is the paramagnetic metal ion, gadolinium. (See Runge et al., 141 Am. J. Radiol. 1209 (1983) (which is hereby incorporated by reference) and Weinman et al., 142 Am. J. Radiol. 619 (1984), which is also hereby incorporated by reference). Although gadolinium shortens both the T1 and T2 times, at the lower doses used for clinical imaging, the T1 effect generally predominates and the image becomes brighter. Also, the RF pulse sequence can be programmed to accentuate T1 changes and diminish those due to T2 (Runge et al., 141 Am. J. Radiol. 1209 (1983)(which is hereby incorporated by reference)). Hence, "T1-weighted" enhancement can be achieved by selecting the most favorable Gd dose and RF pulse sequence.

The shortening of proton relaxation times by Gd is mediated by dipole-dipole interactions between its unpaired electrons and adjacent water protons. The effectiveness of Gd's magnetic dipole drops off very rapidly as a function of its distance from these protons (as the sixth power of the radius). (See Brown, 3 Mag. Res. Imag. 3 (1985)(which is hereby incorporated by reference)). Consequently, the only protons which are relaxed efficiently are those able to enter Gd's first or second coordination spheres during the interval between the RF pulse and signal detection. This ranges from 105 to 106 protons sec$^{-1}$. (See Brown, 3 Mag. Res. Imag. 3.) Still, because Gd has the largest number of unpaired electrons (seven) in its 4f orbital, it has the largest paramagnetic dipole (7.9 Bohr magnetons) and exhibits the greatest paramagnetic relaxivity of any single element (Runge et al., 141 Am. J. Radiol. 1209 (1983), and Weinman et al., 142 Am. J. Radiol. 619 (1984)). Hence, Gd has the highest potential of any element for enhancing images. However, the free form of Gd is quite toxic. This results in part from precipitation at body pH (as the hydroxide). In order to increase solubility and decrease toxicity, Gd has been chemically chelated by small organic molecules.

In mid-1988, a first-generation, low-molecular-weight, paramagnetic contrast agent, gadolinium-diethylenetriaminepentaacetate (Gd-DTPA) dimeglumine, was approved for general clinical use (Berlex-Schering AG; West Germany patent filed by Gries, Rosenberg and Weinman: DE-OS 3129906 A 1 (1981)). This agent has significantly improved the assessment of brain and renal tumors. Nevertheless, its small molecular size causes it to: 1) diffuse freely into normal tissues as well as pathologic foci, thereby decreasing the magnitude and gradient of image contrast at tumor margins; and 2) backdiffuse rapidly from the tumor matrix into blood capillaries, resulting in a very short postinjection contrast interval of 10-45 minutes. This latter characteristic precludes the premedication of patients outside the imaging room. The presence of an intact blood-brain barrier in the normal tissue surrounding brain tumors, reduces the problem of normal brain enhancement by Gd-DTPA.

Despite its satisfactory relaxivity and toxicity, this formulation has four major disadvantages.

(1) Chelation of Gd markedly decreases its relaxivity (by half and order of magnitude). This happens because chelators occupy almost all of Gd's inner coordination sites, which coincide with the strongest portion of the paramagnetic dipole. (See Koenig, 2 Proc. Soc. Mag. Res. Med. 833 (1985)(which is hereby incorporated by reference), and Geraldes et al., 2 Proc. Soc. Mag. Res. Med. 860 (1985)(which is hereby incorporated by reference)).

(2) Gd-DTPA dimeglumine, like all small paramagnetic metal chelates, suffers a moderate decrease in relaxivity at the higher radio frequencies used clinically for proton imaging (typically 15 MHz). (See Geraldes et al., 2 Proc. Soc. Mag. Res. Med. 860.)

(3) Due to its low molecular weight, Gd-DTPA dimeglumine is cleared very rapidly from the bloodstream (1/2 in 20 minutes), and also from tissue lesions (tumors). (See Weinman et al., 142 Am. J. Radiol. 619 (1984)). This limits the imaging window (to ca. 10 to 45 minutes); limits the number of optimal images after each injection (to ca. 2); and increases the agent's required dose and relative toxicity.

(4) The biodistribution of Gd-DTPA is suboptimal for imaging of body (versus brain) tumors and infections. This is due to its small molecular size. Intravenously administered Gd-DTPA exchanges rapidly into the extracellular water of normal tissues, as well as concentrates in tumors and infections. This is facilitated by the absence, in body organs, of the "blood-brain" vascular barrier which partly restricts the exchange of Gd-DTPA into the extracellular water of normal (versus diseased) brain. The result, in body organs, is a reduced difference in the concentration of Gd-DTPA between normal and diseased regions of tissue, and hence, reduced image contrast between the normal and diseased regions of the organ. Also, a disproportionate quantity (>90%) of Gd-DTPA is sequestered very rapidly in the kidneys. (See Weinman et al., 142 Am. J. Radiol. 619 (1984)). Of much greater interest to body MRI, are the abdominal and musculoskeletal-soft tissue sites involved in the early detection and staging of tumors (particularly body tumors, including the liver, spleen, bone marrow, colon, pancreas, and limbs).

Attempts have been reported to conjugate paramagnetic (principally gadolinium) chelates to protein carriers (principally albumin), by adding reactive chelate precursor molecules (principally DTPA anhydride) with the externally exposed amino groups of the intended carrier protein. However, due to the limited number of such amino groups on naturally occurring proteins, and the difficulty of forming amine conjugates, these protein carriers have suffered from low derivitization (substitution) ratios.

Comparably low substitution ratios (normalized for molecular weight) have been reported for immunoglobulins. (See Lauffer et al., 3 Mag. Res. Imaging 11 (1985)(which is hereby incorporated by reference), and Brady et al., 1983 Soc. Mag. Res. 2nd Ann. Mtg., Works in Progress, San Francisco, CA). Comparably low substitution ratos have also been reported for fibrinogen. (See Layne et al. 23 J. Nucl. Med. 627 (1982)(which is hereby incorporated by reference)). This results from the relative difficulty of forming amide bonds, the comparatively low number of exposed amino groups on typical proteins which are available for coupling, and the relatively rapid hydrolysis of DTPA anhydride coupling substrate which occurs in the aqueous solvents required to minimize protein denaturation during conjugation. (See Hnatowich et al., 33 Int. J. Appl. Rad. Isot. 327 (1982)(which is hereby incorporated by reference), and Krejcarek et al., 77 Biochem. Biophys. Res. Comm. 581 (1977)(which is hereby incorporated by refereence)). The overall effect of these suboptimal conditions is that a very large dose of carrier material is required to achieve significant in vivo effects on MR images. At this high dose, the carrier produces an unacceptable acute expansion of the recipient's blood volume by an osmotic mechanism. Indeed, low substitution ratios have generally limited the use of such protein-chelator-metal complexes to the more sensitive(low-dose), radiopharmaceutical applications. (See Layne et al., 23 J. Nucl. Med 627 (1982)(which is hereby incorporated by reference)).

An attempt to overcome this low substitution ratio has been made by conjugating DTPA to the non-protein, cellulose (which is water insoluble). (See Bulman et al., 40 Health Physics 228 (1981)(which is hereby incorporated by reference).) However, the chemical method employed results in continued suboptimal substitution of DTPA to carrier. The nonbiodegradability of cellulose (which is water insoluble) and its water-soluble derivatives, and the reported molecular aggregation which results from organic-solvent conjugation (in dimethylformamide) of CNBr-activated cellulose to the diaminohexyl spacer groups which link the carrier to DTPA, have rendered this class of carrier-conjugates unacceptable for intravenous administration at the doses required for MR image enhancement.

A very important consideration in the image enhancement of solid tumors and inflammatory lesions by polymeric contrast agents is that, in order for these agents to extravasate (exit) efficiently from the microcirculation into adjacent diseased tissues, they must be completely soluble. For example, they must not be contaminated by intermolecular or supramolecular microaggregates. Optimal tumor access and localization requires that the molecular size of such agents generally be less than approximately 2,000,000 Daltons (ca. 2 to 3 nanometers in molecular diameter), and preferably less than 500,000 Daltons (ca. 0.5 to 1 nanometer in molecular diameter; see Jain 1 Biotechnology Progress 81 (1985), which is hereby incorporated by reference), and most preferably, as taught by the present application, less than about 40,000 to 45,000 Daltons. For this reason, with rare exceptions (see Example 6, below), the particulate and microaggregate classes of contrast agents (which comprise the liposomes, colloids, emulsions, particles, microsphere and the larger microaggregates, as described below) do not concentrate efficiently in most solid tumors and inflammatory lesions.

Instead, following intravenous administration, these supramolecular-sized agents:

a) are first circulated in the bloodstream for relatively short intervals (2 minutes to 24 hours, depending on size), potentially allowing direct image enhancement of the blood pool (plasma compartment); and

b) are subsequently cleared by specialized (phagocytic) cells of the reticuloendothelial tissues (liver, spleen and bone marrow), potentially allowing selective enhancement of these normal tissues, but producing indirect (negative) enhancement of lesions region these tissues (due to exclusion of the agents from the diseased regions).

Additionally, following installation into the gastrointestinal tract and other body cavities, these particulate and microaggregate classes of agents can produce direct image enhancement of the fluids within these cavities, and thereby potentially delineate mass lesions which encroach upon the lumens and cavities.

Both microspheres and microaggregates are supramolecular in size. The microaggregate class of agents is produced (intentionally or unintentionally) by either a) molecular cross-linking of individual polymer molecules or b) secondary aggregation of previously singlet (soluble) polymers, as induced by charge attraction or hydrophobic bonding mechanisms. It is distinguished from the microsphere class of agents by virtue of its smaller particle size, which ranges from approximately 2,000,000 Daltons (ca. 2 to 3 nanometers in diameter) to 0.1 micrometers ( = 100 nanometers in diameter). It is important to note that microaggregates are cleared by reticuloendothelial phagocytes with significantly less efficiency and rapidity than are microspheres. In general, this property makes microaggregates a less preferred class of agents for visualizing the liver, spleen and bone marrow under ths usual conditions of clinical imaging, for which prompt post-injection contrast enhancement is required.

Gd-DTPA has been entrapped in liposomes in order to selectively enhance images of the reticuloendothelial organs (liver, spleen and bone marrow) and potentially the lungs. (Buonocore et al., 2 Proc. Soc. Mag. Res. Med. 838 (1985)(which is hereby incorporated by reference).) Liver clearance is mediated by phagocytic (Kupffer) cells which spontaneously remove these small (0.05 to 0.1 micron) particles from the bloodstream (Buonocore et al. (1985) 2 Proc. Soc. Mag. Res. Med. 838). (Particles larger than 3 to 5 micron are selectively localized in the lungs, due to embolic entrapment in lung capillaries.) A recent report indicates that the small-sized Gd-liposomes produce effective decreases in liver T1's (as determined spectroscopically without imaging): see Buonocore et al. (1985) 2 Proc. Soc. Mag. Res. Med. 838). Also, insoluble Gd-DTPA colloids have recently been reported to enhance MR images of rabbit livers under in vivo conditions (Wolf et al. (1984) 4 Radiographics 66 (which is hereby incorporated by reference)). However, three major problems appear to limit the diagnostic utility of these devices. The multilamellar, lipid envelopes of liposomes appear to impede the free diffusion of water protons into the central, hydrophobic cores of these carriers, as assessed by the higher doses of Gd required for in vitro relaxivities equivalent to Gd-DTPA dimeglumine (Buonocore et al. (1985) 2 Proc. Soc. Mag. Res. Med. 838). This increases the relative toxicity of each Gd atom.

Even more importantly, these same lipid components cause the carriers to interact with cell membranes of the target organs in a way which leads to a marked prolongation of tissue retention, with clearance times of up to several months. (See Graybill et al., 145 J. Infect. Dis. 748 (1982)(which is hereby incorporated by reference), and Taylor et al., 125 Am. Rev. Resp. Dis. 610 (1982)(which is hereby incorporated by reference).) Two adverse consequences result. First, image enhancement does not return to baseline in a timely fashion. This precludes re-imaging at the short intervals (ca. 1 to 3-weeks) needed to assess acute disease progression and treatment effects. Second, significant quantities of the liposomally entrapped Gd-DTPA may be transferred directly into the membranes of host cells. (See Blank et al. 39 Health Physics 913 (1980)(which is hereby incorporated by reference); Chan et al., 2 Proc. Soc. Mag. Res. Med. 846 (1985)-(which is hereby incorporated by reference).) This can markedly increase the cellular retention and toxicity of such liposomal agents.

The consequences for Gd toxicity have not yet been reported. Protein (albumin) microspheres with entrapped Gd and Gd chelates have been prepared, and have been determined (by the present inventor and others: see Saini et al. (1985) 2 Proc. Soc. Mag. Res. Med. 896) to have only modest effects on T1 relaxivity in vitro. This is because most of the Gd as well as other entrapment materials are initially sequestered in the interior of these spheres, and are released very slowly as the spheres become hydrated (with $t_{1/2}$s of hours). (See Widder et al., 40 Cancer Res. 3512 (1980)(which is hereby incorporated by reference).) This phenomenon has been found by the present inventor to markedly reduce the acute (30-to-90-minute) relaxivity of each Gd atom to approximately 1/10th that of Gd-DTPA dimeglumine. Hence, both the quantity of carrier material and the toxicity of Gd are both unnecessarily high.

Emulsions of insoluble, gadolinium oxide particles have been injected into experimental animals, with significant image-enhancing effects on the liver. (Burnett et al. (1985) 3 Magnetic Res. Imaging 65). However, these particles are considerably more toxic than any of the preceding materials, and are inappropriate for human use.

## Novel Compositions and Methods for Imaging

Because of the significant disadvantages of existing MR image contrast agents, the present inventor has formulated improved, second-generation prototype agents with reduced toxicity, increased selectivity of tumor and organ uptake, as well as a significant potential for enhancing blood flow images.

A very important consideration, as taught by the present application, is that the marker substance should preferably by selectively deposited at the tissue location which is sought to be imaged. Moreover, the present application also contains significant teachings, regarding how closely the paramagnetic marker substance is bound to the polymer, which are believed to provide substantial advantages over previous teachings. The present application provides a novel method for NMR imaging, wherein image contrast is very strongly enhanced by a selective transport method which introduces superparamagnetic material selectively into the desired imaging locations, and specifically into tumor locations. The present application also provides novel compositions of matter which are useful in implementing these methods.

MRI contrast enhancement can be improved moderately in the brain (and greatly in the body, which lacks the brain's tight blood-tissue barrier), by increasing the tumor selectivity of agent uptake.

Injected gadolinium exchanges off of its DTPA chelator at a slow but significant rate *in vivo*. The resulting free gadolinium forms insoluble oxides, clears slowly from the body, and many produce significant side effects. The present application permits major advantages to be gained, by substituting a less toxic, efficiently cleared, highly paramagnetic ion or polyatomic metal-atom complex in an improved delivery process.

Chromium, in the form of $^{51}CrO_4^{-2}$, has been used extensively as a clinical agent for radionuclide labeling of platelets and red blood cells. Hexavalent chromate is converted within the red cell to the $Cr^{+3}$ cation, which binds tightly but not irreversibly to hemoglobin. $^{51}$Chromium elutes from red cells at an average rate of 0.93% per day. It is not reutilized by the body, but it cleared efficiently by excretory pathways. It is also of low toxicity in humans, even at relatively large doses. According to studies, in which neutrophils, tumor-cells and other biological targets have been labeled *in vitro*, $^{51}CrO_4$ has been shown to bind to several cytoskeletal and cytoplasmic proteins (actomyosin as well as hemoglobin), and also to adenine nucleotides. It has also been shown to be nontoxic *in vitro* at relatively high concentration, as assessed by sensitive measures of cellular metabolism, DNA synthesis and cell division.

When tested as a potential MRI contrast agent, chromium (+3) has only moderate potency compared to gadolinium (+3), which has the highest number of unpaired electrons (7) of any metal ion. On this basis, the low-molecular-weight gadolinium chelate, Gd-DTPA, was developed as the first clinical MRI contrast agent, even though its small retained fraction (usually less than 0.5%) is substantially more toxic than equivalent quantities of retained chromium, and chromium is cleared much more completely than is gadolinium.

## Superparamagnetic Compound

One class of highly paramagnetic compounds is those in which each molecule includes multiple highly magnetic ions with parallel spin vectors. While such intramolecular paramagnetic coupling does not implay that macroscopic ferromagnetic behaviour will occur, it does imply that the resulting compound will be very strongly paramagnetic. Thus, such compounds are referred to as "superparamagnetic."

A recent article by Bino et al., "[$Cr_4S(O_2CCH_3)_8(H_2O)_4$]: Ferromagnetically Coupled $Cr_4S$ Cluster with Spin 6 Ground State", in the 9/16/88 issue of Science at page 1479, reported that the paramagnetic potency of chromium can be increased markedly by reformulating it as an intramolecularly ferromagnetically coupled cluster of four coordinated chromium ions, $Cr_4S(O_2CCH_3)_8(H_2O)_4^{+2}$. This new, divalent chromium-organic complex cation has 12 unpaired electrons (1.7 times as many as gadolinium), and a magnetic spin of S = 6 (ground state). The effective magnetic moment of each $Cr^{+3}$ atom in the molecule is increased, due to stabilization of a coordination state which minimizes intramolecular antiferromagnetism.

The Bino et al. article refers to using the disclosed cation as "spin label". (Spin labeling studies are normally *in vitro* studies.) The Bino et al. article also notes that the water ligands on the cation are potentially labile, so that the hydration sites would provide ligand bonding sites.

This tetra-chromium-sulfur-acetate complex is very advantageous for use in formulating MRI contrast agents of high potency and low toxicity. However, its small molecular size would cause it to equilibrate freely with the total extravascular (plasma + extracellular) water (as does Gd-DTPA), thereby reducing its potency and tumor selectivity.

## Transport Properties of Polymeric Carrier

The present application teaches that major advantages can be gained by complexing or conjugating a superparamagnetic complex, such as $CR_4S(O_2CCH_3)_8(H_2O)_4^{+2}$, to polymeric or microspheric carriers which restrict its biodistribution, increase its selectivity of tumor localization, and amplify its proton relaxivity by slowing its rotational correlation time.

The present inventor has disclosed, in earlier filings, a method for increasing both the chemical potency (proton relaxivity) and tumor selectivity of paramagnetic contrast agents (including Gd-DTPA), by conjugating them to water-soluble, biocompatible carbohydrate polymers (including dextrans), whose molecular size distribution ranges from just above the cutoff for filtration out of normal microvessels (ca. 15,000 Daltons) to just below the cutoff for rapid renal clearance (ca. 45,000 Daltons).

## Optimal Size Range

Selectivity of tumor uptake is conferred by the polymeric size, in conjunction with characteristic changes in microvascular surface properties and an increase in porosity of malignant tumor microvessels. This allows the polymeric species (between 15,000 and 45,000 Daltons) to filter or become transported more efficiently out of microvessels into the extravascular compartment (tumor interstitium). Due to the low porosity of normal microvessels, polymeric contrast agents are not allowed to filter into the surrounding normal tissues. This property of selective partitioning by molecular size: 1) advantageously results in steeper contrast gradients and increased intensity differences between tumor and normal tissues, and 2) advantageously produces highly discrete identification of tumor margins.

## Endothelial Binding

Tumor localization is also facilitated by the endothelial binding properties of polycarboxylated and polysulfated polyglucoses and polyols, including polyglycerols. (Simple polysaccharides will normally not adhere to the endothelial wall, unless the polysaccharide includes a charged surface group, such as sulfate, carboxyl, or dicarboxyl.)

It is newly recognized in this filing that such endothelial binding is reversible and, moreover, the release of such bound materials occurs much more slowly from tumor microvascular endothelium than from the endothelium of normal tissues. Such prolonged binding at pathologic foci results in selectively accentuated uptake into the tumor interstitial gel proximal to sites of vascular endothelial binding.

## Transendothelial Migration

Active endothelial transport has been demonstrated for small molecules (e.g., glucose and insulin). However, no studies other those that of the present applicant are known to have shown such transport for larger molecules, or for molecules carried in a cargo format. It is now known (from the present applicant's histologic studies) that transendothelial migration of particles and molecular aggregates (larger than ca. 2 nm in diameter) can be accelerated by the application of appropriate surface coatings, preferably glycosaminoglycans or anionic polyglucoses or polyglycerols. (The glycosaminoglycans preferably include heparin, heparin derivatives and heparin fragments, but may also include dermatan sulfate, chondroitin sulfate, and other nature or modified glycosaminoglycans, including semisynthetic carboxylated glycosaminoglycans.) These surface coatings will bind multiply to receptors or antigens, which are either synthesized by endothelium or, although synthesized at other sites, become tightly associated with the endothelial surface. (See Ranney, 35 Biochem. Pharmacology 1063 (1986), which is hereby incorporated by reference). Such multiple binding typically involves complementary molecular interactions at more than 5 binding sites per molecule, and preferably more than 10 sites per molecule, and is termed adhesion, surface adhesion, or bioadhesion.

Following extravasation, these polymeric agents percolate through the tumor interstitial gel at a rate about 3 to 8 times slower than do small molecules, including Gd-DTPA. This causes the polymeric agents to be retained for prolonged intervals in the tumor interstitium (greater than 2.5 hours, as compared to about 10-45 minutes for Gd-DTPA), and to remain ("stay put") preferentially in the viable (versus necrotic) subregions. These two properties: 1) allow the agent to be injected at earlier times before imaging (and hence allowing premedication outside the imaging room); 2) permit tumor-treatment effects to be monitored in responding tumor subregions at early post-treatment intervals (at about 6-30 hours); and 3) allow viable

8

and nonviable tumor to be distinguished at submillimeter resolution. This is because dead subregions cease to perfuse (and hence cease to take in the image-enhancing agents), while viable subregions continue to perfuse and take up image-enhancing agent. Partially damaged subregions continue to perfuse, and, since their microvessels typically have a still-further increased porosity (due to treatment-induced primary or secondary vascular damage), they allow the larger species of polydisperse polymer (as well as the smaller ones) to extravasate into the tumor gel. Hence, the partially damaged and potentially recoverable subregions achieve the brightest image intensity, because they accumulate the greatest quantity of polymeric contrast-enhancing agent.

A further new teaching of the present application is that these anionic glycosaminoglycans, polyglucoses and polyglycerols (and analogous compounds) undergo accentuated uptake by tumor cells, compared to the rate of uptake by normal cells in the same tissue region. This is based on the anionic (negatively charged) nature of side groups present on the polyglucose carriers, which engage the cellular uptake receptors of the anionic transport channels (pores) which are typically induced in hepatocellular carcinomas (hepatomas). Such anionic transport channels have also been found in several other tumor types tested to date (by the present applicant and others). These same transport channels are relatively uninduced in the normal cell counterparts. This property of anionic small molecules and macromolecules facilitates active tumor-cell accumulation of the carrier polymer (and its bound ligands) *in vivo*. This property is exploited, in the innovative method disclosed herein, to allow for prolonged tumor retention and imaging.

The following list sets out some of the major advantages of polymeric MRI contrast agents, as exemplified by Gd-DTPA-dextran(40,000, 60,000, or 70,000 Dalton carrier size):

1. Increased proton relaxivity, due to the slower rotational correlation time of polymeric versus small molecular formulation (about 4 to 7 times more T1 relaxivity than that of Gd-DTPA dimeglumine).

2. Restricted biodistribution *in vivo* (in about 10% versus 35% of body water - due to improved retention of polymers versus small molecules in the plasma compartment of normal tissues). This advantage in turn results in:

    a. a decreased dose of paramagnetic metal, and

    b. decreased toxicity (due to decreased normal tissue uptake).

3. Improved body and brain imaging, due to improved selectivity of tumor uptake. This helps to allow detection and monitoring of very small tumors (1-2 mm$^3$).

4. Improved image contrast gradient and magnitude at tumor margins. This too helps to allow detection and monitoring of very small tumors (1-2 mm$^3$).

5. Prolonged tumor imaging enhancement (greater than $2\frac{1}{2}$ hours, versus 10-45 minutes for Gd-DTPA dimeglumine). This prolonged enhancement of the tumor image allows:

    a. patient premedication outside the imaging room (which helps to maximize utilization of the very expensive imaging equipment); and

    b. acquisition of multiple sequential optimally contrast-enhanced images with different pulse parameters, and

    c. imaging of multiple body regions after a single dose of agent.

6. Complete solubility. This provides the advantages of:

    a. allowing rapid renal clearance (if less than 45,000 Daltons), and

    b. avoiding acute uptake by normal liver (unlike Gd-DTPA, which undergoes acute hepatic uptake at about 5-45 minutes, thereby obscuring the visualization of liver tumors during optimal imaging intervals, especially when using T1-weighted pulse sequences).

7. Lower osmolality at typical injection concentrations (e.g. about 285-330 mOsmols per kg of water, at an injection concentration of 100 mg/ml).

8. Acute (20 to 30-hour post-treatment) monitoring of tumor responses can be performed *in vivo* at submillimeter resolution. This is not possible with low molecular weight agents such as Gd-DTPA, because of their very rapid percolation through the tumor gel, which obscures the functionally important living and dead regions.

9. Rapid conjugation, of multiple (e.g. 25 to 100) paramagnetic ions per protein molecule, can occur, to antibodies and to other proteins and peptides. (Some example include: lymphokines (including interleukin 2); cytokines (including tumor necrosis factor and interferons); and other biopharmaceutical agents.) This conjugation occurs by linking the entire polymeric MRI contrast-enhancing agent to the antibody, protein, or peptide, using simple chemical reactions, such as Schiff-base and water-soluble carbodiimide reactions. This provides sufficient amplification (due to the paramagnetic or superparamagnetic signal effect) for the (polypeptide) protein molecule to be detected by MRI contrast enhancement, even at the very low tissue concentrations of protein which are typically achieved in tumor interstitium (about 1 to 2 micromolar).

It has previously been shown, by the present inventor as disclosed in a previous filing (International Application PCT/US88/01096), that IMFERONTM (which is a tightly bound, iron oxide-dextran complex of about 110,000 Daltons) achieves increased intramolecular paramagnetism (becomes superparamagetic), in a fasion similar to that of $Cr_4S(O_2CCH_3)_8(H_2O)_4^{+2}$. This complex is injected into patients for the purpose of achieving controlled iron release, over intervals of days to weeks, in order to treat iron deficiency anemia. Although it has been injected intravenously into patients, this must be done by controlled rather than bolus infusion, due to the release of a small fraction of its ionic iron which has been associated with acute toxicities. Hence, IMFERONTM is usually administered intramuscularly. These problems, together with the requirements, in MR image enhancement, of rapid intravenous administration of relatively large doses of the contrast agent, have precluded the effective use of IMFERONTM as an intravascular superparamagnetic contrast agent. However, this experimental evidence provides further confirmation that, as described below, metal coordinates of high potency and lower toxicity can be reformulated as polymeric agents(with a conjugation chemistry which is somewhat analogous to that of IMFERONTM). Such metal-coordinate-polymer agents can be administered for purposes of tumor-selective MR image enhancement, or alternatively to provide localized hysteresis superheating.

The preferred embodiments preferably use a biodegradable, water-soluble polymer (synthetic or derived from natural sources) which has repeating hydrophilic monomeric units (preferably carbohydrate or sugar residues) with a high frequency of hydroxyl (and/or, in certain embodiments, amino or sulfate) side groups. This polymer also includes additional charged functional groups, (complexing, chelating, or coordinate-forming functional groups), which may include (but are not limited to): amine; quaternary ammonium or other reactive nitrogen group; hydroxyl; carbonyl; aldehyde; carboxy; polycarboxy; sulfhydryl; sulfate; sulfonium; phosphate; polyphosphate; phosphonium; or other homo- or heteroanions. These charged (or chelating or coordinate-forming) functional groups have a formation constant for divalent or trivalent metal cations (or for the organo-metallic complexes which contain these divalent or trivalent metal catios) of at least about 108 (and typically more) at physiological temperature and pH. The conjugation of chelating groups to the polymer (or to form the copolymer) is carried out under chemical conditions and in a solvent which yields a completely soluble (singlet) form of the carrier and avoids significant contamination by microaggregates. The molar ratio of chelating agent/monomeric unit is preferably between about 1/5 and about 1/25. The molar ratio of chelating agent/monomeric unit is preferably between about 1/5 and about 1/25. This image-enhancing agent is biodegradable to intermediary metabolites, rapidly excretable chelates, polymers, oligomers, monomers or combinations thereof, all of which have low toxicity and are cleared overwhelmingly by the renal route. The term "low toxicity" used herein means having little significant toxic effects at usable dosages of the image-enhancing agents.

## Use of Microaggregate or Microparticulate Carrier

In one class of embodiments, the polymeric carrier is used in the form of microspheres. As discussed above, these microspheres have been found (when appropriately surfaced with sites complementary to endothelial determinants) to transport through the more porous parts of the endothelium walls with high preference. This is particularly advantageous in transporting a relatively high dose of the desired substance into the abnormal tissue cells.

Such a microsphere is most preferably between 0.2 and 250 micron in diameter. The matrix of the microsphere is preferably a carbohydrate, and may be a carbohydrate such as heparin which also has multivalent binding capabilities. Dextran can also be used, and can also be coated with a multivalent binding agent such as heparin. Such a microsphere carbohydrate matrix can optionally include, as a multivalent binding agent, an exposed or covert lectin (or peptide, polypeptide, or drug substance) which is capable of binding endothelial surface determinants, enzymes, epiendothelial or subendothelial substances. (Note that the microsphere matrix may be coated with such a binding substance.)

In such embodiments, the microspheres of the novel material disclosed herein will bind to endothelia (or to epithelia dn their closely associated extracellular structures), with preference (and longer residence times) in the vicinity of tumors (or other biological lesions if desired). This preferential binding leads to preferential induction, since a bound microsphere may be totally or partially enveloped in, for example, less than 10 to 15 minutes. The interaction of the preferred microspheres with endothelia may produce an induction of the endothelia to undergo transient separation or opening. The opening of the endothelia exposes underlying substances to which (ideally) binding may occur.

The present application provides improved methods and compositions of matter for the selective tumor localization of ferromagnetically coupled image-enhancing agents, contrast agent or spectral shift agents.

This permits improved acquisition of tumor, tissue or organ images or spectra from live animals by nuclear magnetic resonance imaging or spectroscopy.

## Additional Novel Compositions of Matter

It should be noted that the present application describes not only a number of novel methods, but also a number of novel compositions of matter, as set forth in greater detail below.

One novel teaching of the invention involves use of (I) a ferromagnetically coupled, multiply paramagnetic ion cluster (hereafter also designated the "superparamagnetic complex") which is multiply associated, by complexation (including ion pairing) or covalent conjugation, with (II) a soluble, hydrophilic, biocompatible, excretable polymeric carrier, comprising repeating hydrophilic monomeric units, or with (III) monomeric or oligomeric subunits of the final polymer, which, when combined with the paramagnetic ion cluster, associates with this cluster to form an "in-chain" heteropolymer, and wherein the polymer or polymer subunits (either derived from natural sources of synthetic) have repeating monomeric units with a high frequency of hydroxyl, carbonyl, aldehyde, carboxyl, sulfate, sulfonate, sulfonium, phosphate, phosphonate, phosphonium, amine, amino, or quaternary ammonium groups, singly or in combination on the polymer, and the polymer has a molecular diameter of less than about 12 nanometers, and contains less than about 5% (w/w) cross-linked or microaggregated species, all of low toxicity. The latter groups are for the purposes of either noncovalently binding the superparamagnetic complex or binding to target (including tumor) microvascular endothelium, or binding to both of the preceding entities.

The polymeric agent may optionally be formulated using an excipient counterion to achieve charge balance. Such excipient agents may include, for example, organic amines, preferably including N-methylglucamine (meglumine).

The superparamagnetic complex of the primary preferred embodiments uses a central tetrahedrally coordinated sulfur atom, surrounded by four octahedrally coordinated Chromium atoms, which are stabilized by bridging ligands (which join pairs of Cr atoms). In the embodiment of Example 10, eight bridging ligands are used, and they are all acetate groups. However, in other embodiments, other bridging ligands, and/or a different number of bridging ligands, may be used.

## Alternative Carrier Compositions

The polymers most preferably used are heparin (or heparan sulfate), DTPA-hydroxyethyl-starch (DTPA-HES), or DTPA-dextran. However, of course, a large variety of other carrier polymers could be used instead. Note that the preferred polymer molecules are hydrophilic, which is required to provide the necessary environment for reliable NMR results.

Some of the other preferred polymer species include other dextrans, dextran sulfate, dextran carboxylate, dermatan sulfate, chondroitin sulfate, pentosan polysulfate hydroxyethyl starch, carboxylated hydroxyethyl starch or CARBETIMER™, carboxylated hydroxyethyl starch, and carboxylated dextrans in which the carboxylating groups consist essentially of multiple closely spaced carboxylates which are thereby capable of undergoing chelation-type or coordination-type binding with divalent or trivalent metal ions, or with polyatomic organometallic complex structures which include these metal ions.

The locations of the charged group in the polymer can be readily modified, by methods well known to those skilled in the art, e.g. by introducing succinylate or glutarylate groups to extend the charge ion groups out from the polymer based structure. Thus, where it is desired to increase the affinity of the polyatomic unit being transported for the polymeric carrier molecule, the conformation of the polymer can optionally be modified in this fashion to achieve a better fit.

## Alternative Bridging Ligands in a Superparamagnetic Complex

The bridging ligands in the superparamagnetic complex need not be limited to acetate groups. A wide variety of organocarboxylates may be used. Some examples of alternative bridging ligands include: formate; formaldehyde; glutaraldehyde; glycinate; succinate; acetylacetonate; malonate; propionate; glutarate; hydroxamate; oxalate; 2-bromoacetate; 2-sulfoethanoate, thiolacetate; and thioglycolate.

## Use of Reactive Bridging Ligands

The embodiment described below, which includes at least some glycinates as bridging ligands, has the advantage that the glycinates contain sites which can assist in binding. Thus, a further secondary teaching is that the bridging ligand should contain a charged and/or activatable site.

## Alternative Paramagnetic Species

The paramagnetic ion which is used in the superparamagnetic complex is most preferably chromium, but may alternatively be one or more of the following species: iron, nickel, manganese, cobalt, vanadium, molybdenum, tungsten, copper, platinum (particularly $^{195}$Pt), erbium, gadolinium, europium, dysprosium and holmium.

## Alternative Stabilizing Anions

The superparamagnetic complex $Cr_4S(O_2CCH_3)_8$ is preferably stabilized with water, so that the full formula of this complex cation is $Cr_4S(O_2CCH_3)_8(H_2O)_4{}^{+2}$. However, other stabilizing species can be used, such as sulfate, halide, nitrate, or other stabilizing anions. (Note that some of these anions will be displaced when the complex binds to the endothelia or epithelia.)

## Additional Novel Methods

The present application also sets forth a generally applicable method for selective transport of a desired small polyatomic structure into tumors, or other regions of enhanced vascular porosity. Note that these novel teachings can be applied not only to the method of magnetic resonance imaging described, but also to a tremendous variety of other diagnostic and therapeutic uses.

## Improved Hyperthermia Methods

The present application also provides improved methods for the selective tumor or tumor-cell localization of hyperthermia agents. The present application also provides a method for inducing selective hyperthermia in tumors or tumor cells, with reduced damage to healthy tissue, by microwave hysteresis superheating of tumor-localized agents. In this class of embodiments, the fraction of hyperthermia agents which has not localized in abnormal tissue is not likely to induce other localized hyperthermia, since almost all of this remaining fractions will be on or very close to blood vessel walls, i.e. will be located in regions which are efficiently cooled.

For example, one disclosed embodiment uses the selective transport mechanism to provide selectively localized hysteresis heating. In this embodiment, polymer-encapsulated transportation of superparamagnetic substances is used to achieve selective deposition in tumorous tissue. The high density of paramagnetic material in the tumorous tissue results in a greatly enhanced cross section for absorbing RF energy. Therefore, when RF energy is applied to induce heating, the tumorous regions will be preferentially heated, as is desired. Thus, the cancerous cells can be harmed with minimal damage to healthy tissue.

Similarly, the novel selective transport method disclosed can also be used to transport substances which will intensify X-ray, radionuclide, or ultrasonic imaging.

## Improved Therapeutic Methods

In a further alternative class of embodiments, the disclosed novel transport methods can be used to preferentially transport chemotherapeutic substances into a tumor, as described below.

## BRIEF DESCRIPTION OF THE DRAWING

The present invention will be described with reference to the accompanying drawings, which show important sample embodiments of the invention and which are incorporated in the specification hereof by reference, wherein:

Figure 1 shows a perspective view of a sample superparamagnetic complex which can be used in one embodiment of the disclosed method.

Figure 2 schematically shows a nuclear magnetic resonance imaging system suitable for use in the disclosed innovative method.

Figure 3 is a schematic representation of the transport of microspheres entirely across endothelial (or epithelial) tissue.

Figure 4 is a photograph of stained tissue showing localization of nanospheres. The nanospheres, at the scale of this photograph, appear as small gray round or oval dots of 1 to 2 $\mu$m in diameter.

Figures 5A-5E show infrared data pertinent to the fabrication of a further alternative superparamagnetic complex, which can be used instead of that shown in Figure 1.

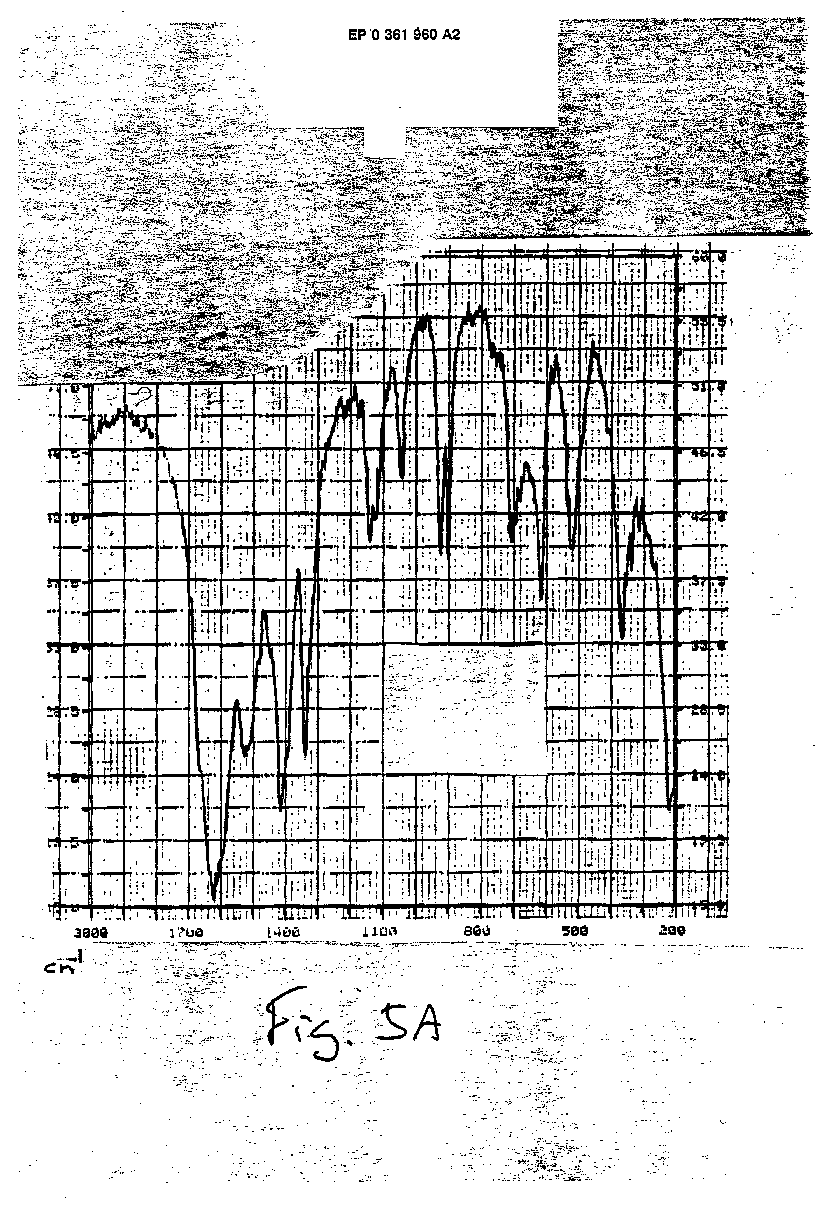

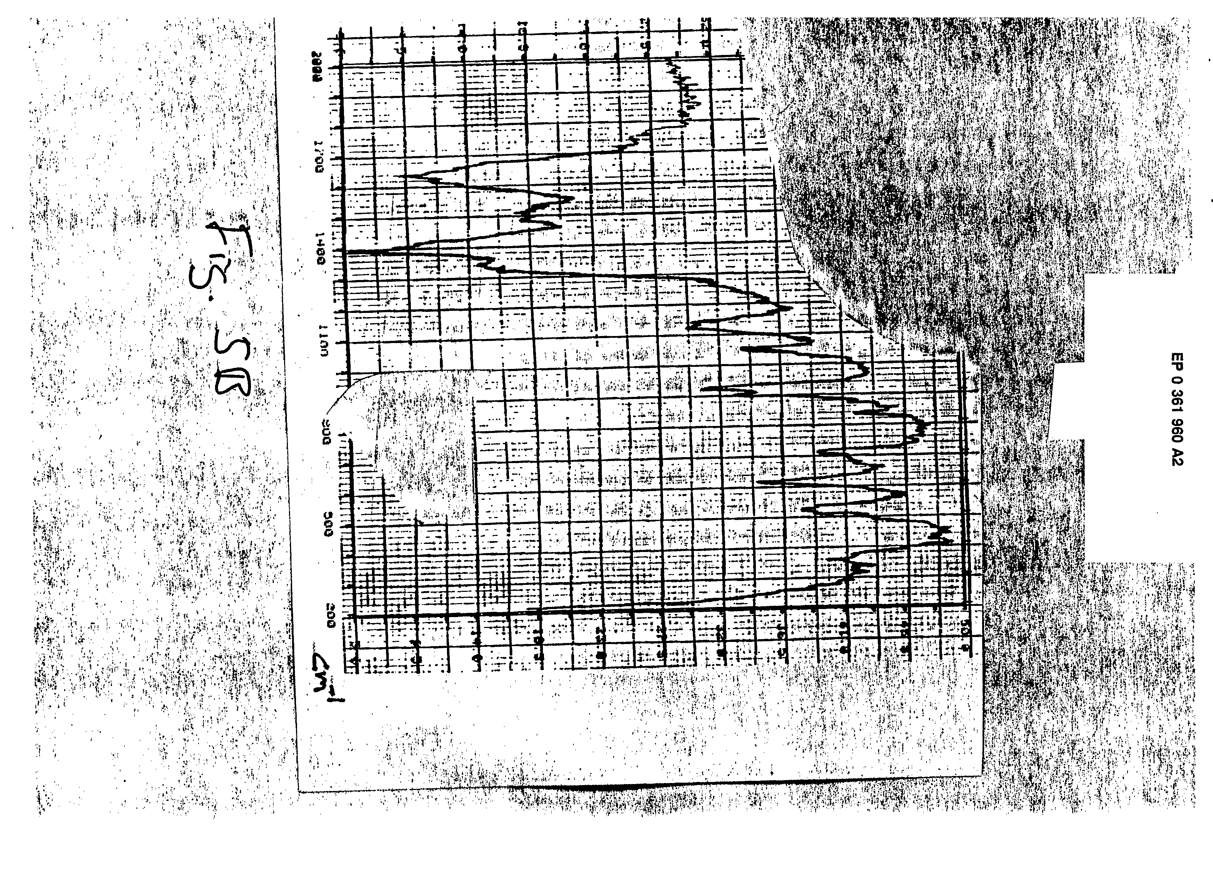

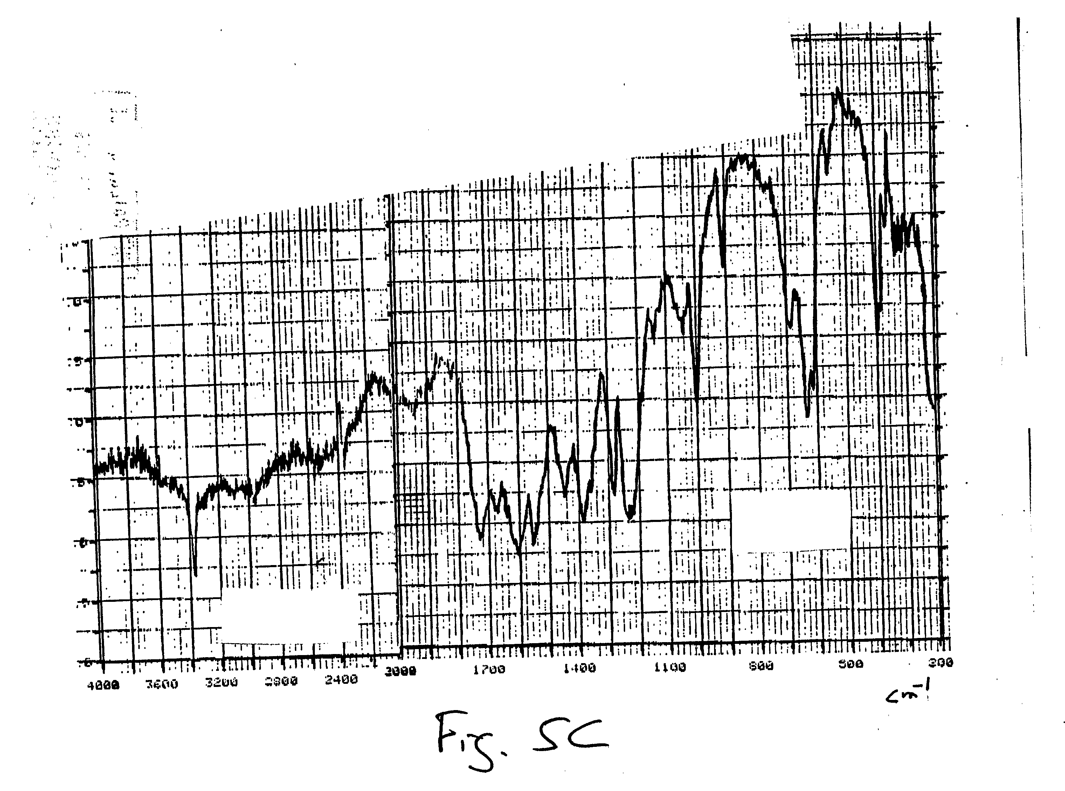

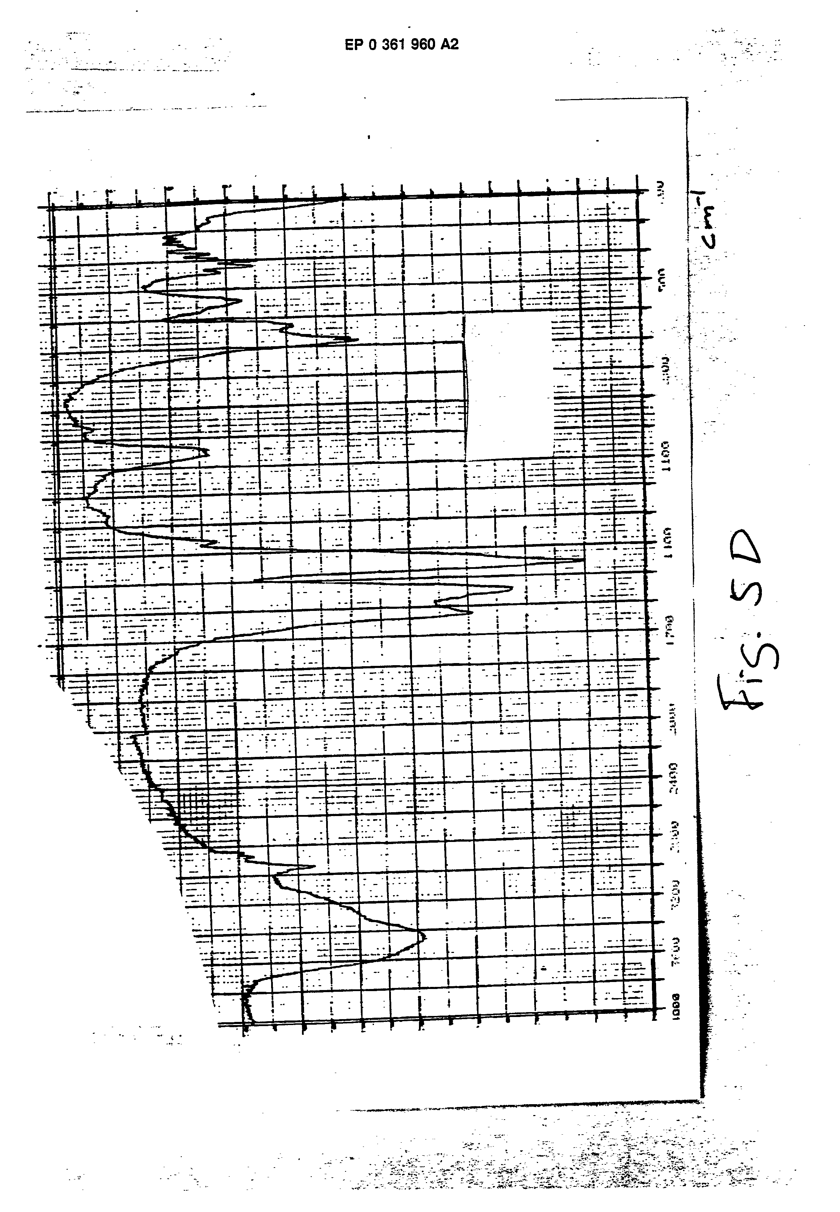

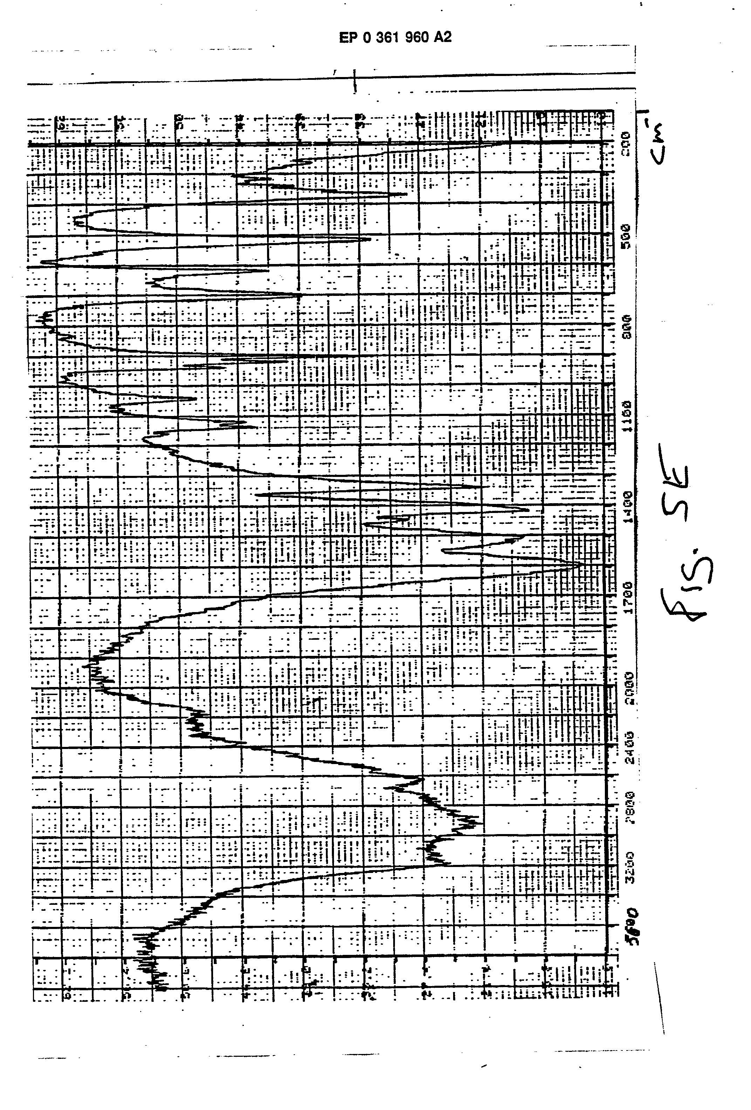

Figure 5A shows an infrared spectrum trace for the reaction products where $Cr_4S(O_2CCH_3)_8$ was heated with glycine in water at 92°C.

Figure 5B shows a trace for the reaction products where $Cr(NO_3)_3$ was heated with glycine in water at 92°C.

Figure 5C shows a trace for the reaction products where $Cr_4S(Ac)_8$ was refluxed in acetic anhydride for severals.

Figure 5D shows a trace for $Cr_4S(Ac)_8$ alone (which has a blue or green color), and

Figure 5E shows a trace for glycine alone.

## DESCRIPTION OF THE PREFERRED EMBODIMENTS

The numerous innovative teachings of the present application will be described with particular reference to the presently preferred embodiment, wherein these innovative teachings are advantageously applied to the particular problems of NMR imaging by selective transport of superparamagnetic small molecules. However, it should be understood that this embodiment is only one example of the many advantageous uses of the innovative teachings herein. For example, the various types of innovative methods disclosed herein can optionally be used to transport acoustic image enhancers, or therapeutic substances. In general, statements made in the specification of the present application do not necessarily delimit any of the various claimed inventions. Moreover, some statements may apply to some inventive features but not to others.

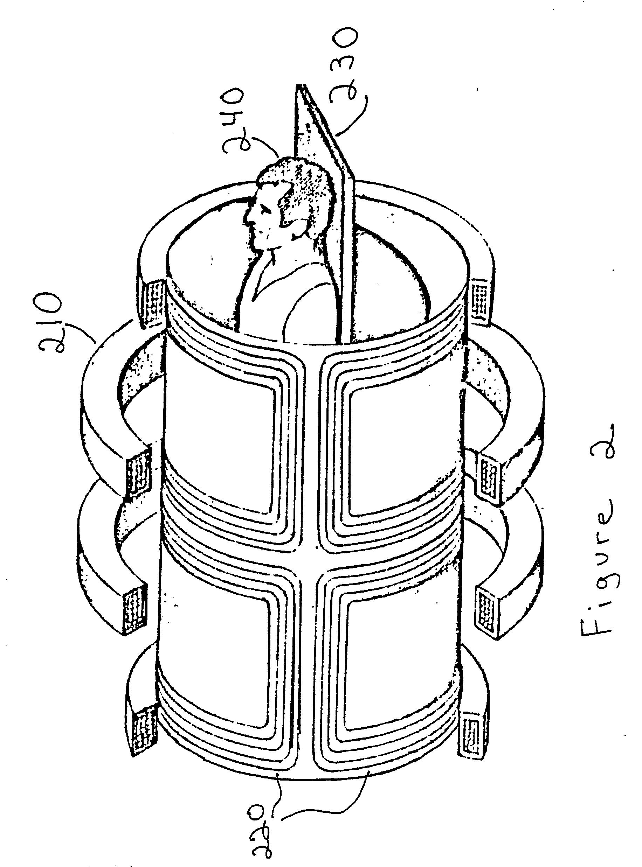

Figure 2 shows an example of a nuclear magnetic resonance imaging (MRI) unit, useable for whole-body imaging of human patients. A patient 240 is inserted, on a sliding table 230, into the interior of a large solenoidal winding 210. The large coils 210 (which may be water-cooled or superconducting)will apply a constant (DC) magnetic field, typically 0.5 to 1 Tesla. (This field component is referred to as the $B_0$ field.) Bias coils 220 apply a gradient to this field, as described above. Finally, a probe coil (which is movable, and is not shown in this Figure) is used to apply the RF pulses described above. Differently shaped probe coils are used for imaging different parts of the body, and the probe coil is often shaped so that it will nearly fit to the shape of the surface of the area to be imaged.

The present invention provides an improved NMR imaging method, whereby the ability of NMR systems to detect tumors is greatly enhanced. This is accomplished by selectively introducing an image-enhancing, spectral shift responsive agent into the abnormal tissue.

One novel teaching of the invention involves use of (I) a ferro-magnetically coupled, multiply paramagnetic ion cluster (hereafter also designated the "superparamagnetic complex") which is multiply associated by complexation (including ion pairing), coordination, or covalent conjugation; with (II) a soluble, hydrophilic, biocompatible, excretable polymeric carrier, comprising repeating hydrophilic monomeric units; or with (III) monomeric or oligomeric subunits of the final polymer, which when combined with the paramagnetic ion cluster, associates with this cluster to form an "in-chain" heteropolymer, and wherein the polymer or polymer subunits (either derived from natural sources of synthetic) have repeating monomeric units with a high frequency of hydroxyl, carboxyl, carbonyl, aldehyde, sulfate, sulfonate, sulfonium, phosphate, phosphonate, phosphonium, amine, amino, or quaternary ammonium groups, singly or in combination on the polymer; and the polymer has a molecular diameter of less than about 12 nanometers, and contains less than about 5% (w/w) cross-linked or microaggregated species, all of low toxicity. The latter groups are for the purposes of either noncovalently binding the superparamagnetic complex or binding to target (including tumor) microvascular endothelium, or binding to both of the preceding entities. The polymeric agent may be formulated using an excipient counterion to achieve charge balance, which may include

13

organic amines, preferably including N-methylglucamine (meglumine). Preferred polymers include heparin, heparan sulfate, dextrans, dextran sulfate, dextran carboxylate, dermatan sulfate, chondroitin sulfate, pentosan polysulfate, hydroxyethyl starch, carboxylated hydroxyethyl starch or CARBETIMERTM, and especially heparin, carboxylated hydroxyethyl starch and carboxylated dextrans in which the carboxylating groups consist essentially of multiple closely spaced carboxylates which are thereby capable of undergoing chelation-type or coordination-type binding with divalent or trivalent metal ions, or with polyatomic organometallic complex structures which include these metal ions.

The ferromagnetically coupled paramagnetic complex includes a molecular coordination compound containing a paramagnetic metal ion present in numbers of two or more per molecular coordinate, wherein the paramagnetic metal ion includes one or more of the following: chromium, iron, nickel, manganese, cobalt, vanadium, molybdenum, tungsten, copper, platinum (particularly $^{195}$Pt), erbium, gadolinium, europium, dysprosium or holmium; and the coordinated metal ions are stabilized in a ferromagnetic or superparamagnetic intramolecular complex configuration by an external complexing substance. The preferred metal ions include chromium and gadolinium, and the preferred external complexing substances includes organosulfates and their derivatives, carboxylic acids, and especially acetate ions. The molecular coordinate may also include a central multivalent stabilizing ion or element, in which case the preferred element includes sulfur. The ferromagnetically coupled, paramagnetic complex (superparamagnetic complex) preferably has a net nuclear spin of greater than about 3/2 and has more than about 7 unpaired electrons. It has labile water groups and a net electrical charge, or reactive groups or combining sites which allow it to chemically associate, covalently or noncovalently with the carrier polymer.

In operation, time-domain windowing may be performed in the NMR imaging run, so that the atoms with the shortest relaxation times are seen preferentially. Alternatively, a high pulse repetition rate may be used, so that the tissues with the longest relaxation times are kept in saturation.

One preferred group of features of the complex for chemical association includes carboxylate, oxygen, metal (especially chromium "hydroxide") glycine amine, and net cationic (positive) charge.

One preferred embodiment involves providing the superparamagnetic coordinate $Cr_4S(O_2CCH_3)_8(H_2O)_4^{+2}$ as a noncovalent complex with heparin of about 22,000 Daltons, wherein the superparamagnetic coordinate binds via its cationic charges to the negatively charged sulfate groups of heparin at a molar ratio of about 1 superparamagnetic coordinate to every 2 to 25 monomeric units of the heparin and all or part (or none) of the excess free sulfate groups are balanced by ion pairing with N-methylglucamine. An alternative preferred embodiment includes an analogous formulation, wherein the superparamagnetic coordinate is complexed with low-molecular-weight hydroxyethyl starch (of less than about 50,000 Daltons) which also carries covalently bound diethylenetriamine pentaacetic acid (DTPA) side groups at a molar ratio of about 1 side group for every 5 to 25 monomeric units. Another alternative preferred embodiment includes an analogous formulation, wherein the superparamagnetic coordinate is complexed with dextran of 40,000 or 60,000 Daltons, which also carries covalently bound diethylenetriamine pentaacetic acid (DTPA) side groups at a molar ratio of about 1 side group for every 5 to 25 monomeric units. In alternative preferred embodiments of this class, the superparamagnetic complex binds to the sulfate groups of dextran sulfate or pentosan polysulfate.

The heparin superparamagnetic paired-ion complexes just described, are preferred for selective uptake by lung, lung tumors and other lung lesions, for MR image enhancement, or hysteresis hyperthermia, following intravenous injection, or for uptake by solid tumors and other focal disease following selective arterial administration. Lung uptake can be increased by leaving a sufficient fraction of heparin's sulfate groups, preferably greater than 30%, unbalanced by counterions. Lung uptake can be reduced and systemic access increased by balancing more completely the negative sulfate groups of heparin with either or both of the superparamagnetic complex or the excipient counterion, including N-methylglucamine.

The DTPA-dextran and DTPA-hydroxyethylstarch (DTPA-HES) complexes with the superparamagnetic substance just described are preferred for selective uptake, MR image enhancement, or hysteresis hyperthermia at systemically distributed sites of disease, including tumors, when administered by either intravenous or intraaterial injection. This is based on reduced lung clearance of the less acidic carboxylic acid (versus sulfate) side groups, which reduces binding to normal (versus lesional, including tumor) endothelium. It is also based on the lower degree of molecular complementarity of DTPA-dextran and DTPA-HES polymers with their endothelial binding substituents, which comprise heparan sulfates.

A second preferred embodiment involves providing the superparamagnetic coordinate, $Cr_4S(O_2CCH_3)_8(H_2O)_4^{+2}$, as a covalent complex with a biocompatible water-soluble excretable carbohydrate polymer, especially dextran of about 40,000 or 60,000 Daltons, or to hydroxyethyl starch of less than about 50,000 Daltons, wherein the superparamagnetic coordinate binds via a metal oxide bond fo chromium to the hydroxyl groups of dextran or hydroxyethyl starch at a molar ratio of about 1 superparamagnetic coordinate to every 2 to 25 monomeric units of the carbohydrate. An alternative preferred embodiment includes an analogous covalent conjugate of $Cr_4S(O_2CCH_3)_8(H_2O)_4^{+2}$ to carboxylated carbohydrate polymers, especially to DTPA-dextran of about 40,000 or 60,000 Daltons, or to DTPA-hydroxyethyl starch of less than about 50,000 Daltons, wherein the superparamagnetic coordinate binds via a metal-oxide bond of chromium to the carboxyl oxygen of the DTPA side groups on the polymer.

In certain instances, it may be preferable to separate the bound superparamagnetic substance from the polymeric carrier, using a linker which has a chain length of preferably between about 4 and 8 carbon atoms. This may be desirable under circumstances in which a) the superparamagnetic substance is a bulky molecule, or b) it is important to stabilize the bond between the polymer and the linker against lysis, including hydrolysis and esterolysis, which may in unusual cases, be catalyzed by a substituent of the superparamagnetic substance.

In an alternative embodiment, a method of modifying the spatial relation of the magnetic substance to carboxylated carbohydrate polymers, is specified. This alternative method involves derivatizing the carbohydrate polymer with a higher bifunctional acid, including preferably succinic acid, to form a succinylated polymer with a 4-carbon spacer between the polymer and the superparamagnetic to be conjugated to the polymer via the free carboxylate group of each succininate linker.

Although the preceding preferred methods of conjugation have focused on ester and carboxylate linkages, other linkers are not excluded, and in some cases may be preferred. These include aldehyde, amine, amide, carbodiimide, halogen-activated carbohydrate groups, and combinations thereof.

A third preferred embodiment involves providing a physical microsphere or nanosphere form of the preceding agents in which the diameter of the spheres ranges from 0.1 to 250 micrometers, and the particles are preferably formed from their paired, polymer-superparamagnetic ion complexes or from simple mixtures of the polymer and the substance to be entrapped. Matrix polymers and excipients preferably comprise 50-75% of the particle weight. The particles are prepared preferably by phase emulsification (for larger ones) or high-pressure homogenization (for smaller ones), followed by heat or chemical stabilization of the polymer matrix, and extraction of the oil phase with an organic solvent, including acetone, ether, or hexane, preferably acetones which may also contain a small quantity of biocompatible detergent for surface stabilization, preferably Tween 80 (or deoxycholate) at about 0.05 to 0.5% (w/w). Smaller particles are provided by high pressure homogenization. The degree of heat or chemical stabilization will preferably determine how long the particle retains its physical form following rehydration for *in vivo* administration, and will also determine how rapidly the internally entrapped superparamagnetic substance is made available, hydrated, exposed or released, in order that it can modify the biochemical environment of the plasma, extracellular matrix (or matrix water), or intracellular cytoplasmic substituents (or water). Preferably heat stabilization of carbohydrate matrices is performed for about 30 seconds to 5 minutes, in order to render the matrix sufficiently stabilized that the entrapped material becomes chemically exposed over an interval of about 15 minutes to 30 hours. For both the induction of MRI contrast and the amplification of hysteresis heating the $t_{1/2}$ for release of entrapped agent occurs preferably within about 15-20 minutes of injection, although under certain circumstances, particularly those involving the monitoring of controlled-release drugs from selectively localized microcarriers, this $t_{1/2}$ may be considerably longer. Also, for MRI contrast enhancement, the entrapped material must be hydrated (released) in order to affect surrounding diffusible water protons, whereas, for hysteresis heating, the entrapped material need not be released at all, but can function while still entrapped, providing that the macrodomain size of an average superparamagnetic deposit within the particle is sufficiently large for efficient hysteresis to occur, preferably larger than about 0.5 micron. Otherwise, release of entrapped substituent with subsequent reconcentration by extracellular matrix binding or cellular processes, is preferred in order to achieve an efficient hysteresis response in the tissues.

The smaller particle sizes (of less than 3 micrometers, and especially less than 0.8 micron) are prefered for systemic administration by intravenous injection and for selective arterial administration into critical end-arterial circulations. The larger particle sizes of greater than 5 micrometers, and especially greater than 100 micrometers are preferred for chemoembolization of selected organs with blood supplies, including especially the liver, by selective arterial administration, and for introduction mechanically, directly into tumor masses or body cavities.

The acute enhancement of blood flow (or perfusion) images, for example in the heart of cerebral vessels, may be accomplished with the soluble polymeric image-enhancing agents and is even more efficiently performed with the nanosphere and microsphere forms.

A significant advantage of MRI enhancement with polymeric, nanosphere and microsphere superparamagnetic substance, is a further reduction of the dose and any potential toxicity over that which can be achieved by simple (low molecular weight) superparamagnetic substances alone.

The relatively rapid biodegradation and metal clearance times, and the resultant shorter reimaging intervals are particular advantages involved with the present invention relative to other polymeric and particulate metal oxides, chelates and complexes.

The image-enhancing agents of the present invention, in soluble or microsphere form, are readily reconstituted for animal and patient administration. This reconstitution involves a simple vortex-type mixing, as contrasted with the sonication in detergents used for protein-based microspheres.

The image-enhancing agents of the present invention are easily usable in any MRI detection system involving administration of paramagnetic or ferromagnetic contrast agents. It has particular advantages in conjunction with the newer rapid RF pulse sequences, which reduce native tissue contrast in order to shorten image acquisition times and increase patient throughput. The image or spectral enhancing agents of the present invention allow shorter image acquisition times for satisfactory internal resolutions. These times are generally adequate to produce satisfactory internal images because of the greater enhancement and image contrast produced per unit of superparamagnetic and total agent.

The potential for selective localization of large numbers of relatively nontoxic superparamagnetic molecules by small number of monoclonal antibodies, nonpeptide and peptide hormones, lymphokines, cytokines, and other receptor-binding substances tagged with one or more of the innovative (and preferably polymeric) image-enhancing agents is contemplated as a major diagnostic advancement for future use.

The potential for selective localization of large numbers of relatively nontoxic superparamagnetic molecules by small numbers of carrier polymers, nanospheres or microspheres is contemplated as a major therapeutic advantage for future use in conjunction with hyperthermia augmentation by hysteresis heating and delivery and monitoring of tagged therapeutic agent localization in sites of disease.

Because of the high MRI contrast conferred by these superparamagnetic substances and the substantial prolongation of lesional residence times, use of the present image-enhancing agents will allow an increased number of serial images to be obtained in an enhancement mode after a single administration of agent.

Due to the selective retention of the carriers used to formulate the present image-enhancing agents, superior definition of tumor margins and markedly improved discrimination of viable and nonviable tumor subregions is possible. This has the major advantage of allowing tumor responses to chemotherapy and radiation therapy to be monitored at early posttreatment times and submillimeter resolution, several weeks before small tumor nodules would regrow to volumes detectible by computerized axial tomography (CAT) and radionuclide scanning.

From a chemical standpoint, some advantages of the present invention may be summarized as follows. When MR image-enhancing agents comprise superparamagnetics, each superparamagnetic substance exhibits an increased relaxivity for adjacent magnetic nuclei (e.g., protons) and hence, gives greater T1 signal enhancement. This increased relaxivity is related to an increased dipolar correlation time of the superparamagnetic substance due to its slower molecular rotation when polymerically controlled. Spacer groups are not required between the superparamagnetic substance and the polymeric carrier in order to obtain optimal paramagnetic relaxation potencies, however, they could be introduced if deemed advantageous for other purposes.

The chemically defined nature of preferred chelator-polymer combinations allows ready batch-to-batch uniformity for improved pharmaceutical formulations and a likely greater ease of regulatory approval.

Many of the preferred polymers of the present invention, such as certain dextrans (40,000 and 70,000 MW forms), hydroxyethyl starch, and heparin, for example, have already separately achieved final regulatory approval for patient administration. The size of these polymers is optimized to prevent access into normal tissues, but to still allow rapid renal clearance and essentially complete body clearance. Also, due to the association of multiple, potent superparamagnetic substances with each polymer molecule, the resulting complexes and conjugates comprise low osmolality agents by comparison to their low-molecular weight counterparts. Such low osmolality agents have been shown to have major advantages in several categories of high-risk (particularly cardiovascular) patients.

For parenteral administration, these agents are preferably formulated as a sterile, physiologically balanced, aqueous solution (or suspension), whose pH for purposes of intravenous administration is 6.5 to 7.0. Alternatively, these agents may be lyophilized and provided in the dried form for reconstitution in physiologic solutions just prior to administration. For injection into body cavities (such as the bladder, uterus, Fallopian tubes, nasal sinuses or ventriculo-cerebrospinal system), these agent may be formulated as a physiological solution (or suspension) which contains additional substances (excipients) to increase the viscosity or osmolality. Other additives and formulations may also be incorporated according to standard pharmaceutical procedures.

For parenteral administration, the concentration of total active agent (polymer-superparamagnetic substance) will be between about 0.1% and 30% (weight/volume), typically between about 5% and 25%, and preferably about 20%. Doses of the soluble polymer, nanosphere and microsphere agents will vary depending on the superparamagnetic substance used and the route of administration. The following doses are given for intravenous administration. For tumor image enhancement with some of the preferred embodiments, which include soluble $Cr_4S(O_2CCH_3)_8(H_2O)_4^{+2}$ complexed to DTPA-hydroxyethyl starch, DTPA-dextrans or heparin, the dose of chromium (and sulfur) will be between about 0.005 and 0.025 millimoles per kilogram of body weight, with optimal image enhancement occurring at or below about 0.01 millimoles per kilogram. FFFor enhancement of the cardiovascular blood pool, the optimal dose of preferred soluble agents will occur at or below about 0.04 and 0.02 millimoles of superparamagnetic per kilogram.

For hysteresis heating, the nanosphere or microsphere forms of agents will be administered once or multiply, at about 15 minutes to 2 hours prior to each treatment by either systemic intravenous injection, direct administration into superficial tumors, or by intraarterial perfusion, at doses of up to 1.0 mmol/kg of the superparamagnetic substance. Hysteresis hyperthermia at a frequency of about 10 to 150 kHz will be directed from an external oscillating magnetic source whose maximal energy displacement is centered over the major external or imageable mass(es) of tumor. A major anticipated advantage of using the nanosphere or microsphere form of preferred embodiment, is that a high concentration of clustered (macrodomain) metal is selectively localized in the diseased subregions of the target tissue following transvascular administration, and this localized material further concentrates in the viable, most heavily perfused subregions of tumor which require the greatest augmentation of heating to compensate for their disproportionate loss of heat due to blood flow dissipation.

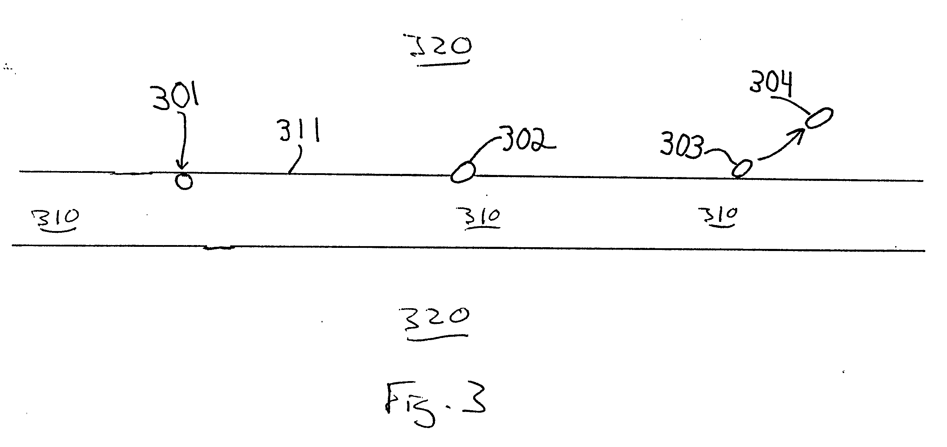

Figure 3 schematically shows how the trans-epithelial transport of microspheres works. A small blood vessel 310 is shown passing through tissue 320. Four microspheres (or nanospheres) 301, 302, 303, and 304 are shown at different stages of passage.

Microspheres 301 has recently adhered to the endothelial wall 311. Thus, this microsphere is said to be at the stage of endothelial adhesion.

Microsphere 302 is at the stage of endothelial envelopment. This will occur after a few minutes, as the wall 311 gradually covers a particle (like microsphere 301) which has adhered to it.

Microsphere 303 is shown at the further stage of extravasation, after envelopment has passed it entirely across the endothelial (or epithelial) wall 311.

Finally, microsphere 304 is shown at yet a further stage, of percolation through tissue.

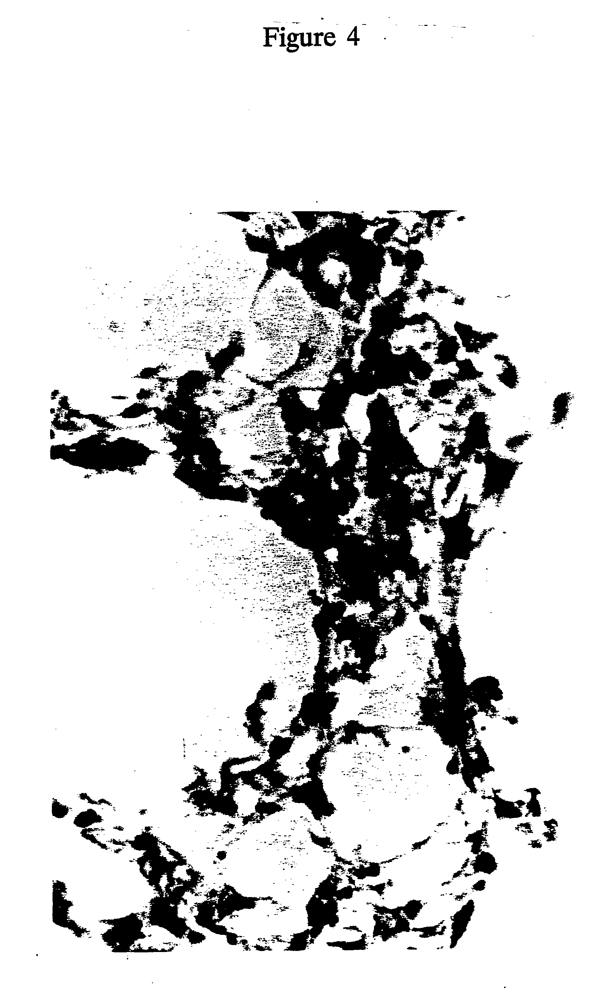

Figure 4 is a photograph of stained tissue showing lung localization of heparin-coated subembolizing (0.1 - 0.8 micron) nanospheres distributed throughout rodent lung tissue at 5 minutes after intravenous injection via the tail vein. The nanospheres, at the scale of this photograph, appear as small gray round or oval dots of 1 to 2 mm in diameter.

The following examples are presented to illustrate preferred embodiments of the present invention and their use in MR imaging or hysteresis heating. These examples are purely illustrative, and do not in any way delimit the full scope of the present invention.


EXAMPLE 1


PREPARATION OF DTPA-DEXTRANS


The cyclic dianhydride of DTPA (diethylenetriamine pentaacetic acid), as prepared by the method of Eckelman et al. (J. Pharm. Sci. V 64, pp 704-706 (1975), was obtained in a highly pure form from Calbiochem-Bhering Corp. The completely soluble DTPA derivative of dextran was prepared by adding 7.0 g of the cyclic DTPA dianhydride stepwise to 1.72 g of Dextran T70 (average MW 70,000 Daltons, $M_N$ 46,000, Pharmacia Chemicals) in a reaction solvent comprising HEPES buffer 115 mg/100 cc distilled water, pH 7.0 to 8.0. The reaction was carried out with vigorous stirring at ambient temperatures for 1 hr with readjustment to pH 7.0 using NaOH, after the addition of each aliquot of DTPA dianhydride.

The dextran-DTPA product was separated from unconjugated DTPA by dialysis against 200 volumes fo 0.15 N NaCl and then 50 volumes of distilled water at pH 6.5. This and the following step are major improvements over the derivatization method previously filed by the present inventor. (See U.S. Patent Applications 799,757 and 086,692, and PCT Application PCT/US86/02479, which are hereby incorporated by reference.) Upon completion of dialysis, the conjugate was brought again to 115 mg/100cc in HEPES buffer, and reacted a second time with an identical quantity of DTPA dianhydride as described above. After this, the dialysis was repeated as described above.

As assessed by molecular filtration, 98% of the dextran-DTPA product had a molecular weight of less than 100,000 Daltons. The dilute solution of DTPA-dextran was either: a) concentrated to between 10% and 25% (w/v) by forced filtered-air evaporation at room temperature, or b) lyophilized to dryness for prolonged shelf storage. Concentrated salts and buffers were added as needed, to render the final preparations physiologically acceptable for injection. The pH was maintained between 6.5 and 7.0. As assayed by complexometric titration, one ligand of DTPA was conjugated for every 7 sugar residues, for a total of 55.5 DTPAs per 389 glucose units in each average molecule.

Two other soluble DTPA-dextran derivatives were synthesized from dextrans of starting molecular weights = 10,000 Daltons (Dextran T10, Pharmacia Chemicals) and 40,000 Daltons (Dextran T40, Pharmacia Chemicals). All of the preceding dextrans were soluble and free of microaggregates, as assessed by filtration through serial molecular sieve filters (Amicon Corporation).

EXAMPLE 2

PREPARATION OF DTPA-HYDROXYETHYL STARCH

Low-molecular-weight hydroxyethyl starch is obtained in a highly pure and soluble form from American Critical Care/DuPont, reacted with the cyclic dianhydride of DTPA, and the polymeric derivative separated, concentrated and titrated as described in EXAMPLE 1.

EXAMPLE 3

PREPARATION OF SUCCINYLATED-DEXTRANS

Succinyl anhydride is obtained in a highly pure form from Aldrich Chemicals, and reacted with dextrans of 40,000 MW and 70,000 MW, and the polymeric derivative separated, concentrated and titrated as described in EXAMPLE 1.

EXAMPLE 4

PREPARATION OF THE PAIRED-ION METAL CO-ORDINATE-POLYMER COMPLEX CISPLATIN-DTPA-DEXTRAN (70,000 MW)

Lyophilized DTPA-dextran (70,000 MW), prepared as in Example 1, was dissolved in 1.4 cc of sterile water, heated for 30 seconds for swirling in a boiling water bath, added (hot) to 70 mg of Platinol™ powder (containing 3.33 mg of cisplatin, $Pt(NH_3)_2-Cl_2$ with the remaining weight comprising excipients; Bristol Laboratories), the solution vortexed vigorously for 30 seconds to dissolve the Platinol™ + excipients, and the resulting solution cooled to room temperature and checked for complete solubility at 2.4 mg/ml. Formation of a stable paired-ion complex between the platinum coordinate and the carboxyl groups bound to dextran, was established by three tests: a) continued solubility of cisplatin at a concentration greater than its native solubility limit of 1.5 mg/cc; b) reduction in the complexation of exogenously added calcium ions by the DTPA groups of DTPA-dextran (assessed using an Orion Instruments ionized calcium analyzer); and c) elimination of tetany following intravenous injection of the resulting mixture into CBA/J mice (Jackson Laboratories) at a dose of 10 mg/25 gm body weight. This absence of tetany contrasts with the occurrence of tetany and death in mice which were injected with an equivalent dose of DTPA-dextran alone - an importantly, in the absence of balancing quantities of calcium ion, which render the resulting Ca-DTPA-dextran entirely nontoxic *in vivo*. Hence, by *in vitro* and *in vivo* criteria, cisplatin (as Platinol™) undergoes complexation to DTPA-dextran at a sufficient binding stability to compete with an added, divalent metal cation ($Ca^{+2}$). The exact coordination state and chemical structure of the resulting cisplatin-DTPA-dextran complex has not been further elucidated.

## EXAMPLE 5

## PREPARATION OF THE PAIRED-ION METAL COORDINATE-POLYMER COMPLEX, CISPLATIN-HEPARIN (22,000-26,000 MW)

Beef lung heparin (Upjohn Company) was added dry at 14 mg to 280 mg of dry Platinol™ powder (Bristol Laboratories) containing 14 mg of cisplatin, the mixture dissolved in 14 cc of sterile water and vortexed for 1 minute to completely dissolve all components. Formation of a stable paired-ion complex between the platinum coordinate and the sulfate groups covalently bound to heparin, was established by two tests: a) continued solubility of cisplatin at a higher concentration (2.25 mg/cc) than its native solubility limit of 1.5 mg/cc; and b) alteration of cisplatin biodistribution following intravenous and intraaterial injection in animals (see the Examples below).

## EXAMPLE 6

## PREPARATION OF HEAT-STABILIZED, HYDROXYETHYL STARCH-MATRIX NANO-SPHERES WHICH ENCAPSULATE CISPLATIN AND HAVE A HEPARIN SURFACE COATING

Hydroxyethyl starch 605 mg (Sigma Chemicals) was suspended in 5.5 cc of sterile water and heated for 3 minutes in a boiling water bath to bring it into a stable (translucent) emulsion, and 5 cc of this was added to 1000 mg of Platinol™ (Bristol Laboratories) containing 50 mg of cisplatin. This was nanoemulsified for 30 seconds in 70 cc of heated (100°C) cottonseed oil (Sargent Welch) using a Brinkmann Instruments ultrasonic homogenizer, and the oil cooled in a room-temperature water bath, with continued homogenization for 2 more minutes, until the emulsion itself reached room temperature. This was extracted 4 times with acetone (Fisher Chemicals) containing 0.5% Tween 80 (Sigma Chemicals), and was harvested by centrifugation and air dried. The resulting particle diameters ranged from 0.1-1.0 micron.

A heparin coating was applied to the particle surfaces by adding 2 cc of a water solution containing 50 mg of beef lung heparin (Upjohn Company), adding the particle suspension plus heparin to 70 cc of heated (100°C) cottonseed oil and repeating the emulsification and extraction steps described in the preceding paragraph. The resulting particles ranged from 0.1 to 0.8 micron in diameter. The presence of a heparin surface coating was verified by suspending the particles in normal saline and adding protamine (Sigma Chemicals), a multivalent heparin-binding agent. This produced aggregation and agglutination of the heparin-coated (but not uncoated) particles.

## EXAMPLE 7

## *IN VIVO* TESTING FOR SELECTIVE LUNG LOCALIZATION OF THE PRECEDING PREPARATIONS FOLLOWING INTRAVENOUS ADMINISTRATION

CBA/J mice (Jackson Laboratories) were injected intravenously via the tail vein with a) nanoparticles containing a heparin surface (as in Example 6) and b) soluble heparin-cisplatin complex-es (prepared as in Example 5). At 5 to 15 minutes postinjection, the animals were sacrificed, their lungs removed and fixed with intratracheal buffered formalin, tissue section cut at 8 microns thickness, and the sections stained using a newly devised method for platinum which comprises a microwave-augmented iron-type stain ($60°C \times 2$ minute x 3 cycles) using a 1:1 mixture of 2% ferriferrocyanide reagent and 4% HCl. By this method, lung uptake of both the nanospheres (see Figure 4) and soluble paired-ion complex of cisplatin-heparin was documented at the 5-minute postinjection interval. Rapid uptake occurred in both extracellular and intra-cellular compartments, and additional histochemical positivity of bronchial respiratory epithelium and paratracheal lymph nodes was observed at 10-15 minutes. No significant staining was observed following intravenous injection of a standard formulation of Platinol (Bristol Laboratories). To those skilled in the art, additional evidence for selective lung localization, was obtained by injecting intravenously, analogous (subembolizing) heparin-coated nanoparticles containing encapsulated amphotericin B, into identical mice, homogenizing the lungs, and documenting an 8-fold increment in drug levels over native amphotericin B (deoxycholate formulation, Fungizone; Squibb) recovered at 1 to 3 hours postinjection. (See U.S. Patent Application No. 07/033,432, and PCT application PCT/US88/01096, which are hereby incorporated by reference.) Hence, the preceding histologic stains correlated with an increment of nearly 1 order of magnitude in selective pulmonary carrier and drug localization.

## EXAMPLE 8

## IN VIVO TESTING FOR SELECTIVE TUMOR LOCALIZATION OF THE PRECEDING PREPARATIONS FOLLOWING INTRAARTERIAL ADMINISTRATION

Additional documentation for maintenance of the cisplatin-carrier paired-ion complex *in vivo* was obtained as follows. Rabbits bearing VX2 carcinomas of the right hind limb were catheterized under fluoroscopic control, and three of the preceding Platinol formulations, as well as standard Platinol, were injected at a constant dose of 15 mg (of cisplatin) per rabbit by selective arterial perfusion over 15 minutes, into the tumor-bearing limb. Animals were sacrificed at 15 minutes, and the tumors and organs were homogenized extracted and analyzed by atomic absorption for tissue platinum concentrations, as shown in Table 1.

Table 1

| Platinum content (ng/mg of tissue, wet weight) - Ipsilateral | | | | | |
|---|---|---|---|---|---|
| Agent | Blood | Tumor | Muscle | Liver | Kidney |
| Heparin-cisplatin: | 2.71 | 12.24 | 0.18 | 5.74 | 5.88 |
| DTPA-dextran cisplatin: | 2.14 | 10.81 | 0.29 | 3.47 | 2.92 |
| Heparin-coated hydroxyethyl starch nanospheres of cisplatin: | 2.43 | 14.07 | 0.20 | 4.64 | 5.91 |
| Standard: | 2.36 | 8.40 | 1.09 | 6.09 | 4.09 |

Additionally, histochemical platinum stains were performed on the tumor tissues, as described in Example 7. These stains revealed intracellular tumor-cell (but not normal-cell) platinum in all of the groups in Table 1 except group 4. Moreover, the intracellular staining of tumor cells in groups 1-3 was significantly more intense than the background staining of hemoglobin iron in red blood cells. Since hemoglobin iron is present at a mean corpuscular hemoglobin concentration (MCHC) of about 0.2 molar, these results suggest strongly that tumor cell platinum reaches very high concentrations relative to those achieved with standard PlatinolTM. To individuals skilled in the art, this also indicates that selective tumor-cell augmentation of hysteresis heating may be achieved by first administering one or more of the carrier formulations described above, but in stable complexation with a strong superparamagnetic substance which is otherwise too small and uncontrolled to undergo this degree of selective localization.

EXAMPLE 9

PROLONGED ENHANCEMENT OF HUMAN TUMOR (MELANOMA) XENOGRAFTS IN NUDE MICE, BY PARAMAGNETIC CHELATE ASSOCIATED COVALENTLY WITH DEXTRAN 70

The strong paramagnetic metal ion, gadolinium ($Gd^{+3}$), was chelated stoichiometrically to DTPA-Dextran 70 polymer, whose preparation is described in Example 1, paragraph 2 (improved formulation). Swiss nude mice were inoculated with BRO-strain human malignant melanomas, and these were allowed to grow to a 1-1.5 cm diameter. Moderately T1-weighted MR imaging (TR = 500 msec, TE = 40 msec) was performed in a standard DiasonicsTM medical imager and a 30-cm RF head coil, before and after intravenous injection of equivalent doses of Gd-DTPA-Dextran-70 (0.03 mmol Gd/kg) or Gd-DTPA (0.1 mmol/kg) contrast agent. Gd-DTPA-dextran and Gd-DTPA began to optimally enhance the tumors at comparably short postcontrast intervals of 10 minutes, however, by one hour Gd-DTPA had completely faded, whereas Gd-DTPA-dextran continued to enhance these tumors intensely for longer than 2.5 hours (the cutoff time on imaging experiments). To those skilled in the art, it will be recognized from these results that chelated Gd and small metal coordinates in general, will benefit greatly in terms of potency (by at least half an order of magnitude), tumor selectivity and tumor retention, from covalent conjugation or strong paired-ion association with dextran or including analogous carbohydrate carrier molecules. It will also be recognized that much lower doses of the stronger superparamagnetics (including doses below about 0.01 mmol/kg, versus 0.1 mmol/kg for Gd-DTPA) can be used to obtain effective MRI contrast enhancement and that much faster, more heavily T1-weighted pulse sequences can be implemented in the presence of superparamagnetic-polymer conjugates or paired-ion complexes.

In the preceding study (see PCT Patent Application PCT/US88/01096), $^{153}$Gd-DTPA-dextran was documented to clear from the blood with a $t_{1/2}$ of about 50 minutes (versus 20 minutes for Gd-DTPA). Total body clearance was almost complete (greater than 96%) by 24 hours. Hence, to those skilled in the art, it will be recognized that strong association of a metal coordinate with a polymeric carbohydrate of predominantly less than 50,000 MW, allows rapid and complete blood and body clearance by predominantly renal pathways.

EXAMPLE 10

PREPARATION OF PAIRED-ION MOLECULAR COMPLEXES OF $Cr_4S(O_2CCH_3)_8(H_2O)_4^{+2}$ WITH NEGATIVELY CHARGED POLYMERIC CARBOHYDRATE CARRIERS

The following negatively charged polymeric carriers are obtained for individual addition and ion pairing to the ferromagnetically coupled paramagnetic cation cluster, $Cr_4S(O_2CCH_3)_8(H_2O)_4^{+2}$ (Exxon Corpora tion)-(see the Bino et al. article cited above): heparin (22,000-26,000 Daltons, Upjohn Company); DTPA-dextrans (40,000 and 70,000 MW parent carbohydrates, derivatized as in Example 1); DTPA-hydroxyethyl starch (50,000 MW parent carbohydrate, prepared as in Example 2); and succinylated-dextrans (40,000 and 60,000 MW parent carbohydrates, derivatized as in Example 3). Each polymer is added as a concentrated aqueous solution, at stoichiometric charge equivalency, or at 50% or 25% of charge equivalency, to a concentrated aqueous solution of $Cr_4S(O_2CCH_3)_8(H_2O)_4^{+2}$ as the chloride salt. Ion pairing is achieved by direct mixing and heating for 1-5 minutes to 100° C at pH 7, with vigorous stirring. To subfractions of the 25% and 50% mixtures (above) is added a concentrated aqueous solution of N-methylglucamine at quantities sufficient to achieve electrical neutrality. The stability of ion pairing is tested by performing equilibrium dialysis against 200 volumes of 0.15 N NaCl and assaying the retained (polymeric) materials for T1 relaxivity (IBM PC20 NMR Spectral Analyzer). Those skilled in the art will recognize from the results of cisplatin complexation to heparin and DTPA-dextran (documented in Examples 4 and 5) that the even more positively charged $Cr_4S(O_2CCH_3)_8(H_2O)_4^{+2}$ counterion of the present example results in an even stronger ion pairing to heparin and DTPA-dextran than the satisfactory (in vitro and in vivo) pairing achieved for the cisplatin metal-amine coordinate documented in Examples 4 and 5.

## EXAMPLE 11

### PREPARATION OF $Cr_4S(O_2CCH_3)_8(H_2O)_4^{+2}$ COVALENTLY CONJUGATED TO NEUTRAL POLYMERIC CARBOHYDRATE CARRIERS

The following two neutral polymeric carriers are obtained for individual addition and ion pairing to the ferromagnetically coupled paramagnetic cation cluster, $Cr_4S(O_2CCH_3)_8(H_2O)_4^{+2}$(Exxon Corporation): dextran and hydroxyethyl starch. Each polymer is added at 50 mg/cc to a saturated aqueous solution of $Cr_4S(O_2CCH_3)_8(H_2O)_4^{+2}$ as the chloride salt, in order to drive the reaction towards conjugation. Reaction is carried out at an alkaline pH of 8 to 10, at 4° C initially (and at room temperature in another run), with mild-to-moderate heating (including 45-60° C) and over 30 minutes to 10 hours (most preferably 1-3 hours) as has been used and documented previously for manufacturing of dextran-iron oxide (ImferonTM, Fisons Corporation - see Cox et al., 208 Nature 237 (1965); Cox and King, 207 Nature 1202 (1965); and Cox et al., 24 J. Pharm. Pharmac. 513 (1972), all of which are hereby incorporated by reference), in order to facilitate formation of the chromate-oxide bond with hydroxyl groups of the dextran or hydroxyethyl starch. The resulting conjugates are dialyzed and tested for stability and NMR T1 relaxivity as described in Example 10. Those skilled in the art will recognize from the ImferonTM literature and from knowledge that the water protons of $Cr_4S(O_2CCH_3)_8(H_2O)_4^{+2}$ are labile (see the Bino et al. article cited above), whereas the chromium-ion cluster is quite stable, thereby allowing formation of a chromate-hydroxyl bond with neutral carbohydrates which have moderate-to-high stability, thereby generating useful covalent conjugates.

## EXAMPLE 12

### PREPARATION OF $Cr_4S(O_2CCH_3)_8(H_2O)_4^{+2}$ COVALENTLY CONJUGATED TO POLYMERIC CARBOHYDRATE-CARBOXYLATE CARRIERS

The following three carboxylated polymeric carriers are conjugated individually to the ferromagnetically coupled para-magnetic cation cluster, $Cr_4S(O_2CCH_3)_8(H_2O)_4^{+2}$ (Exxon Corporation): DTPA-dextran (prepared as in Example 1), DTPA-hydroxyethyl starch (prepared as in Example 2) and succinylated-dextran (prepared as in Example 3). Transesterification conjugation of $Cr_4S(O_2CCH_3)_8(H_2O)_4^{+2}$(as the chloride salt) is carried out by adding the superparamagnetic ion at a saturating concentration individually to the three carboxylated carbohydrates. Reaction is carried out at an alkaline pH of 8 to 10, with mild-to-moderate heating (preferably at 45-80° C) and over 30 minutes to 10 hours (preferably over 1-3 hours), as is accepted practice for transesterification procedures (see Morrison & Boyd, Organic Chemistry (1959). The resulting conjugate is dialyzed and test for stability and NMR T1 relaxivity as described in Example 10. Those skilled in the art will recognize, from the literature on trans-esterification and from knowledge that the water protons of $Cr_4S(O_2CCH_3)_8(H_2O)_4^{+2}$ are labile, that formation of the chromium-carboxylate bond with the carbohydrates acetate or succinylate ligands will occur and have sufficient stability for *in vivo* use, thereby generating useful covalent conjugates.

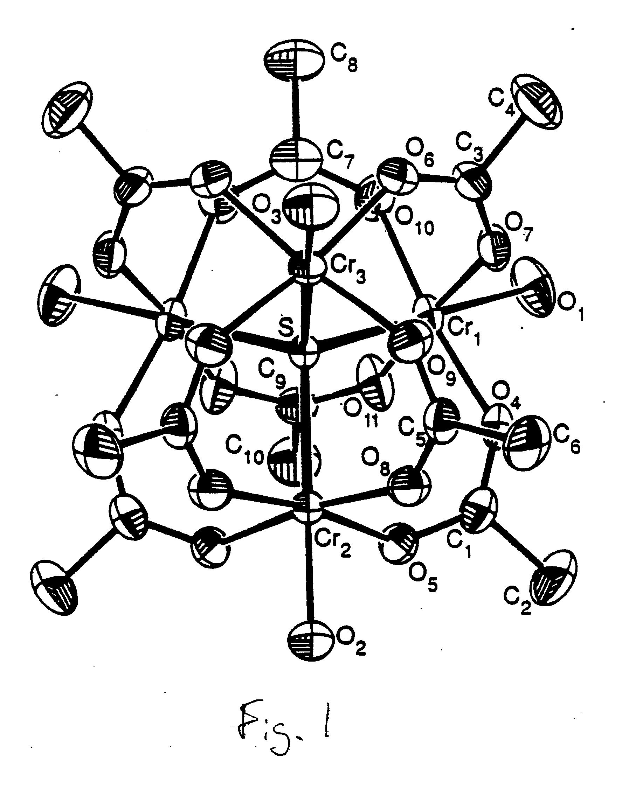

Figure 1 shows a perspective view of the $Cr_4S(O_2CCH_3)_8(H_2O)_4^{+2}$ cation (excluding hydrogen atoms). The atoms are shown with ellipsoids, to indicate approximate thermal vibration ranges at room temperature.

## EXAMPLE 13

### ADVANTAGES TO NMR IMAGING OF POLYMERIC FORMULATIONS OF SUPER-PARAMAGNETICS, INCLUDING FERROMAGNETICALLY COUPLED PARAMAGNETIC COMPLEXES

Selected polymeric formulations of $Cr_4S(O_2CCH_3)_8(H_2O)_4{}^{+2}$, prepared as described in the preceding Examples, are injected intravenously to obtain systemic lesional uptake (frequently practiced in radiology), or intraarterially (less frequently practiced in radiology), to obtain highly selective uptake in regional tumors, especially of the liver, pelvis, brain and limbs. Those skilled in the art will recognize that the more potent, more selective, less toxic (including especially chromium nontoxicity) polymeric formulations of super-paramagnetics, including $Cr_4S(O_2CCH_3)_8(H_2O)_4{}^{+2}$ and analogous cluster compounds incorporating ions such as $Gd^{+3}$ or $Fe^{+3}$, advantageously allow the dose of paramagnetic, including chromium, to be reduced to less than about 0.005 to 0.01 mmol/kg of body weight. (See Ranney, Contrast Agents in Magnetic Resonance Imaging, in Excerpta Medica at page 81 (1986), which is hereby incorporated by reference). It will also be recognized that MRI procedures involving fast imaging (see Bluem et al., 157 Radiology 335 (1985), which is hereby incorporated by reference) and cardiovascular MRI (including MRI "angiography") (see Nagler et al., 157 Radiology 313 (1985), which is hereby incorporated by reference) will benefit greatly, in terms of shortened image acquisition time and improved quality, from a nontoxic contrast polymer of super-paramagnetic potency. Additionally, the polymeric superparamagnetic labels described in the preceding Examples are useful for co-labelling either therapeutic drug carriers (polymeric or nanospheric) or the therapeutic agents themselves, whose tumor (or other lesional) localization needs to be monitored and whose rate of release from the carrier (bioavailability) needs to be assessed noninvasively in vivo, potentially in multiple lesions at different depths within a body region. In this context, polymeric super-paramagnetic labels are useful and of improved utility due to increased potency and selectivity, and reduced toxicity. Furthermore, increased potency allows drug release to be monitored over longer postinjection intervals within target tissues, organs, tumors and infections. These improvements are based on the present application, and also, in part, on applicant's earlier-reported work on the use of partially analogous, but less potent, Gd-DTPA-dextran-labelled drug carriers leading to localization in tissues and enhanced MRI detection. (See the Ranney and Huffaker article at 507 Proc. NY Acad. Sci. 104 (1987), which is hereby incorporated by reference.)

<div align="center">EXAMPLE 14</div>

## ADVANTAGES TO HYSTERESIS HEATING OF POLYMERIC FORMULATIONS OF SUPER-PARAMAGNETICS, INCLUDING FERROMAGNETICALLY COUPLED PARAMAGNETIC COMPLEXES

Microinhomogeneities of tissue heating represent a major problem in hyperthermia treatment of tumors. This results in considerable part, from the selective survival of tumor cells lying adjacent to microvessels -in which heat loss is accentuated by blood flow. A partially effective approach to this problem has been to inject small ferromagnetic particles of $Fe_2O_3$ directly into the tumor masses, and then apply magnetic hysteresis heating at frequencies of 10-100 kHz to the entire local region. (See Borelli et al., 29 Phys. Med. Biol. 487 (1984). Effective superheating and tumor regression in mice occurs if the injected magnetic material is present 1) in sufficient quantity and 2) at a sufficient macrodomain size for efficient hysteresis augmentation to occur (including 0.5-2 micron particle diameters). Because the carrier ion-cluster agents described in the preceding Examples have the properties of markedly improved selectivity and dose of tumor localization, retention in the viable (perfused) subregions of tumor, and improved tumor-cell uptake, it will be understood by those skilled in the art that these carrier ion-cluster agents can be of significant benefit in augmenting the homogeneity, magnitude and tumor-cell selectivity of hysteresis heating induced by oscillating magnetic fields, provided that the associated superparamagnetic agents (which may be associated by conjugation, ion-pairing, or encapsulation) become concentrated as adequately sized macrodomains (of at least about 0.5 micron) in the target sites or cells. Histologic staining for cisplatin (per Example 7) of the VX2 rabbit carcinomas which were perfused with heparin-coated cisplatin-hydroxyethyl starch nanospheres (or 0.1-0.8 micron diameters), documented that many of the tumor cells in the target region addressed by selective arterial perfusion, stained intracellularly in a punctate pattern, wherein the diameters of punctate staining positivity ranged from 0.1 to 0.8 micron ( = the diameters of the original particles). Importantly, high (including about 0.2 molar) intracellular concentrations of cisplatin were achieved in the VX2 carcinoma cells in vivo (see Example 8). The combination of these high levels plus intracellular aggregation were achieved by administering the cisplatin formulated as a heparin-coated microparticle. This documents the type and extent of intracellular accumulation and aggregation of superparamagnetics which are useful in locally amplifying exogenous hysteresis heating. Notably, aggregated staining was absent following intraarterial perfusion of standard (soluble, low-molecular-weight) cisplatin. Hence, in the present example, heparin-coated $Cr_4S(O_2CCH_3)_8(H_2O)_4^{+2}$ hydroxyethyl starch nanospheres and microspheres of 0.8-3.0 micron diameters, when administered intraarterially, are useful as amplifiers of hysteresis heating, and therefore as inducers of augmented cell death *in vivo*.


## EXAMPLE 15


## GLYCINE-SUBSTITUTED NEW POLYATOMIC Cr CLUSTER


The substrate, $Cr_4S(O_2CCH_3)_8(H_2O)_4$, was prepared as described by A. Bino *et al.* (Science, Vol. 241, pp. 1479-1482, Sept. 16, 1988). This was added at 0.5 gm to 50 cc of acetic anhydride, followed by the addition of 0.1 gm of glycine. The mixture was refluxed at 142 degrees C for 4 hours, resulting in a yellow-green solution which was poured while still hot into cold water. Evaporation of the solvent gave yellow crystals, which, when recrystallized, gave an infrared (IR) spectrum indicative of glycine substitution for some of the acetate bridging groups, and retention of Cr-O and Cr-S bonds (Fig. 5C, with Figs. 5D and 5E representing the appropriate controls). Specifically, the IR bands around 1600 cm$^{-1}$ (Fig. 5C) are characteristic of ionized bidentate glycine, and the bands between 300 and 400 cm$^{-1}$ are also consistent with this. The additional bands between 300 and 400 cm$^{-1}$ are also consistent with the presence of Cr-O and Cr-S bonds. Figs. 5A and 5B show the degradation controls wherein the $Cr_4S(O_2CCH_3)_8(H_2O)_4$ degrades to ionic chromium (by IR spectral criteria) when heated with glycine in an aqueous (protonating) solvent, water, for 4 hours at 92 degrees C. These results provide strong evidence for the formation of a novel polyatomic chromium-atom cluster compound of the general formula, $Cr_nS(O_2CCH_3)_x(Gly)_y(H_2O)_z$, wherein n is the number of Cr atoms greater than 1, x is the number of acetates between about 3 and 7, y is the number of glycines between about 1 and 5, and z is the number of loosely bound waters. The inclusion of bidentate glycine as a new bridging ligand in the polyatomic chromium cluster provides a new reactive (charged) group for binding to carriers and renders the entire compound new and novel.


## EXAMPLE 16


## NEW POLYATOMIC GADOLINIUM CLUSTER COMPOUND


Gadolinium chloride (3.7 gm, Alpha) and $Na_2S$ (2.4 gm, Sigma) were mixed in 25 cc of a 1:1 mixture of glacial acetic acid and acetic anhydride and refluxed at 138 degrees C for 3 days. After removal of the dark red, gadolinium-negative filtrate, a lightly tan-colored precipitate was recovered which was qualitatively positive for gadolinium and which, after recrystallization, yielded a water-soluble compound whose IR spectrum was consistent with gadolinium acetate. Importantly, several additional bands were present in the carbonyl (ca. 1500 cm$^{-1}$) and C-O (ca. 1000 cm$^{-1}$) bond stretching wavelengths, which are strongly indicative of a polyatomic (polymeric) nature of this complex and, hence, indicative of a novel, gadolinium-containing, polyatomic complex for use with the disclosed carriers.


## EXAMPLE 17


## FORMATION OF STABLE CARRIER/POLYATOMIC METAL ATOM COMPLEXES


Carboxymethyl dextran was obtained commercially and mixed with $Cr_4S(O_2CCH_3)_8(H_2O)_4$. The result-

ing complex had a T1 relatively (R1) 3 times greater than that of the simple chromium cluster, indicative of strong paired-ion binding between the chromium cluster and the carrier. Analogous mixtures were performed of $Cr_4S(O_2CCH_3)_8(H_2O)_4$ and (a) dicarboxyethyl dextran; (b) heparin; and (c) dextran sulfate. The resulting paired-ion complexes were stable to dialysis in 0.15 molar (isotonic) to 0.5 molar saline.

## Further Modifications and Variations

It will be recognized by those skilled in the art that the innovative concepts disclosed in the present application can be applied in a wide variety of contexts. Moreover, the preferred implementation can be modified in a tremendous variety of ways. Accordingly, it should be understood that the modifications and variations suggested above are merely illustrative. These examples may help to show some of the scope of the inventive concepts, but these examples do not nearly exhaust the full scope of variations in the disclosed novel concepts.

For example, although the presently preferred embodiment is primarily directed to imaging, the selective transport advantages provided could also be used to enhance the performance of NMR spectroscopy of the human body if desired.

For another example, it is alternatively possible to combine a carrier group with a small therapeutic complex. Combinations of boron (or a boroleptic group which provides a site for boron), or of cis-platinum, with a carrier group like those described above many be advantageous. The active agent may be selected to provide chemotherapeutic impact, or to provide sensitization or augmentation for radiation treatment.

For one example, although the disclosed innovations are particularly advantageous in selective transport to tumor sites, they can also be adapted for use with a wide variety of other types of disease of pathology, to selectively address sites where "vascular-permeability-increased" tissue exists. For example, the disclosed innovations can be adapted for use in treatment or imaging (or fine-scale diagnosis) of arthritis, diabetic angiopathy, retinitis, transplantation rejection, or other inflammatory conditions.

Similarly, the disclosed innovative ideas can also be adapted for selectively imaging sclerotic tissue, and thus may be useful in dealing with conditions such as arteriosclerosis or mutliple sclerosis.

For yet another example, the disclosed innovative ideas can also be used to monitor rates of drug arrival, release, or backdiffusion.

In a further alternative, synthetic polymers other than CARBETIMERTM could be used. CARBETIMERTM is a polyaldehyde/polyamine synthetic polymer, which provides useful transport characteristics as a carrier. Many other such synthetic polymers have been proposed, and could be used, if desired, as the polymer in the carrier.

It should also be noted that the carrier can be used either as a polymer or as a microsphere (or other supermolecular aggregation). Polymers are most preferably given a molecular weight in the range of 15,000 to 40,000 Daltons, as described above; but larger polymer sizes may be advantageous for some applications. In particular, where the toxicity is very low (as with chromium), it may be advantageous to use a polymer whose molecular weight is above the renal clearance limit. In such cases, the resulting clearance time will permit the composition to be used as a "blood pool," where a low blood concentration is available over a long period of time to diffuse into a target site. (This may be particularly useful for therapeutic applications.)

Microspheres are even larger than the largest preferred polymer sizes. For example, a polymer of 200,000 Daltons molecular weight will have a typical maximum dimension of less than 12 nm, whereas a microsphere will have a diameter of 100 nm or more. The present invention may optionally be used with microspheres as large as 250 microns. (The larger microsphere sizes are primarily useful for embolization imaging of lung and tumor, and for imaging body cavities, such as lung, bladder, bowel, or central nervous system cavities.) Microspheres may include a surface coating which provides available reactive groups, such as hydroxyl, carbonyl, aldehyde, carboxylate, sulfate, phosphate and amine groups (singly or in combination), for binding to the complex being transported, whereas these reactive groups need not be present in the matrix of the microsphere. However, it should be noted that the polymers of the microsphere matrix should preferably be completely water-soluble, to facilitate clearance from the body.

It should also be noted that a composition of microspheres; with a diameter between about 0.1 micrometer and about 4.0 micrometers, and a polyatomic metal atom cluster which consists essentially of $(Cr_4S(O_2CCH_3)_8(H_2O)_4)^{+2}$ bound to diethylenetriaminepentaacetate-dextran, diethylenetriamine pentaacetate-hydroxyethyl starch, heparin, dextran sulfate or pentosan polysulfate, is believed to be particularly advantageous for liver imaging.

A further point which should be noted is that carriers (such as dextran) have been ionically coupled to an active agent, to produce drug salts; but is has not been conventional to chelate an active agent to a carrier, as is disclosed in some of the innovative examples above. As the examples above show, this further innovative teaching is believed to provide significant advantages.

For superparamagnetic polyatomic structures, it should be noted that heteropolyatomic structures can be used to reduce the need for binding ligands. For example, it is known that vanadium, cobalt, or tungsten can be used as binding atoms to stabilize the relative positions of chromium atoms.

For another modification of the superparamagnetic polyatomic structures, it is expected that the central coordinating atom (which is sulfur in the $Cr_4S(O_2CCH_3)_8(H_2O)_4^{+2}$ example) could alternatively be tungsten, or even vanadium, molybdenum, cobalt, or other species.

In a further alternative, it has been found that transport of glycerol by a polymeric carrier actually increases the permeability of the vascular walls in the tumorous region. Thus, optionally, this effect can be used to further increase the selectivity of delivery of the desired agent.

Moreover, the varying requirements of various applications may imply a rebalancing of the various factors enumerated. For example, where it is desired to transport a metal ion which is very non-toxic, loose binding to the polymeric carrier may be perfectly acceptable. Conversely, in some cases covalent bonding may be particularly advantageous. Paired-ion embodiments may be advantageous for improved renal excretion. Polymers less than about 45K Daltons are particularly advantageous for rapid renal excretion. (Of course, in assessing the size of a polymer composition, it must be recognized that there will normally be a distribution of sizes actually present. The references to molecular weight of polymers herein generally refer to the molecular number weight, or $M_N$, i.e. the peak of this distribution.)

As will be recognized by those skilled in the art, the innovative concepts described in the present application can be modified and varied over a tremendous range of applications, and accordingly, their scope is not limited except by the allowed claims.

Although the invention is being described mainly in connection with diagnostic methods, it also has other uses and may, for example, be used in pathology.

## Claims

1. A composition of matter comprising:
a superparamagnetic **polyatomic complex**, containing plural paramagnetic metal atoms mutually ferromagnetically coupled, and including multiple **bridging ligand** groups which are bound to ones of said paramagnetic atoms,
said polyatomic complex being bound to or associated with:
a biocompatible, clearable **carrier** comprising a substantially completely water-soluble polymer which is native or modified carbohydrate, oligosaccharide, polysaccharide, glycosaminoglycan, or structurally analogous synthetic polymers; said carrier having repeating hydrophilic monomeric units which contain covalently bound available reactive groups selected from the group consisting of one or more of: hydroxyl, carbonyl, aldehyde, carboxylate, sulfate, phosphate and amine groups, singly or in combination;
wherein the molar ratio of said polyatomic complex to said carrier is at least 10:1.

2. The composition of Claim 1 defined further, wherein said reactive groups of said carrier are chemically complementary to determinants of vertebrate endothelia or epithelia.

3. The composition of Claim 1, defined further as an *in vivo* diagnostic agent with the capacity to enhance internal images or shift internal spectra arising from induced magnetic resonance signals.

4. The composition of Claim 1, wherein said carrier has a molecular weight between about 15,000 and 40,000 Daltons.

5. The composition of Claim 1, wherein said polyatomic metal atom complexes are bound to said carrier noncovalently by a strong ionic, paired-ion or charge interaction, and wherein said strong ionic, paired-ion or charge interaction is by chemical coordination or chelation binding of said polyatomic metal atom complex by ones of said reactive groups of said polymer, each site of said coordination or chelation binding having a coordination number of between 2 and 10 inclusive.

6. The composition of Claim 1, wherein the molecular diameter of said carrier is less than about twelve nanometers, and said polyatomic metal atoms complex is in a molar ratio to said monomeric units of said carrier of between about 1:2 and about 1:8, and said carrier contains less than about 5% (w/w) cross-linked or microaggregated species, all of low toxicity.

7. The composition of Claim 1, wherein said water-soluble polymer of said carrier comprises one or more of the following: dextran, dextran sulfate, diethylaminoethyl dextran, carboxymethyl dextran, dicarboxyethyl dextran, succinylated dextran, diethylenetriaminepentaacetate-dextran, hydroxyethyl starch, dicarboxyethylhydroxyethyl starch, carboxymethylhydroxyethyl starch, succinylated hydroxyethyl starch or diethylenetriamine pentaacetate-starch, heparin, heparin sulfate, dermatan sulfate, pentosan polysulfate, dicarboxyethyl heparin, succinylated heparin or diethylenetriaminepentaacetate-heparin, and CARBETIMER™.

8. The composition of Claim 1, wherein said paramagnetic metal atoms include one or more selected from the group consisting of: chromium, copper, manganese, iron, platinum, cobalt, vanadium, molybdenum, tungsten, gadolinium, erbium, dysprosium, europium, and holmium.

9. The composition of Claim 1, wherein said superparamagnetic polyatomic complex has the formula $(Cr_4SR_nC_m)$, where R is a bridging ligand including one or more of: formate, formaldehyde, glutaraldehyde, acetate, glycinate, succinate, acetylacetonate, malonate, propionate, glutarate, hydroxamate, oxalate, 2-bromoacetate, 2-sulfoethanoate, thiolacetate or thioglycolate; n is the number of R groups per metal atom complex, numbering between 4 and 12; C is a stabilizing counterion selected from the group consisting of water or one or more of the following ions: halide, sulfate, nitrate, carboxylate, and phosphate; and m is between 1 and 2n.

10. The composition of Claim 1 defined further, wherein said bridging ligands comprise a combination of acetate and glycinate groups at a molar ratio of between about 1 and 3 glycinates per polyatomic cluster, and amino groups of said glycinate assist binding to said carrier.

11. The composition of Claim 1, wherein said polyatomic metal atom complex comprises a water-soluble water-stable compound in which said metal atoms comprise gadolinium atoms bound to acetate groups.

12. The composition of Claim 22, wherein said microspheres have a diameter between about 0.1 micrometer and about 4.0 micrometers, and said polyatomic metal atom cluster consists essentially of $(Cr_4S(O_2CCH_3)_8(H_2O)_4)^{+2}$ bound to diethylenetriamine pentaacetate-dextran, diethylenetriamine pentaacetate-hydroxyethyl starch, heparin, dextran sulfate or pentosan polysulfate.

13. A composition of matter comprising:
a **metal atom complex** or coordinate, bound by chelation or coordination to:
a biocompatible, clearable **carrier** comprising a substantially completely water-soluble polymer selected from the group consisting of: native or modified carbohydrate, oligosaccharide, polysaccharide, glycosaminoglycan, or structurally analogous synthetic polymers; said carrier having repeating hydrophilic monomeric units which contain covalently bound available reactive groups;
wherein said metal atom complex is bound to said carrier by coordination or chelation binding of said metal atom complex by ones of said reactive groups of said polymer, each site of said coordination or chelation binding, on said carrier, having a coordination number of between 2 and 10 inclusive.

14. The composition of matter of Claim 13, wherein said metal atom complex includes a therapeutic agent which is a metal-atom-containing antitumor drug, cis-dichlorodiamine platinum or other platinum complexes and coordinates, or boron or boroleptics.

15. the composition of matter of Claim 13, wherein said metal atom complex includes cis-dichlorodiamine platinum or other therapeutic platinum coordinates, and wherein said polymer of said carrier is heparin or diethylenetriaminepentaacetate-dextran.

16. A composition of matter comprising:
a **metal atom complex** or coordinate, bound to:
a biocompatible, clearable **carrier** comprising a substantially completely water-soluble polymer selected from the group consisting of: native or modified carbohydrate, oligosaccharide, polysaccharide, glycosaminoglycan, or structurally analogous synthetic polymers; said carrier having repeating hydrophilic monomeric units which contain covalently bound available reactive groups which are complementary to determinants of vertebrate epithelia or endothelia; and wherein said carrier has a molecular weight which substantially always falls between about 15,000 and 40,000 Daltons.

17. A method for magnetic resonance imaging, comprising the steps of:
identifying a living vertebrate animal to be imaged;
introducing into the blood stream or body cavity of said animal a diagnostic imaging or spectral enhancement or spectral shift agent comprising a hydrophilic polymeric compound which comprises a carrier molecule with a molecular weight of greater than about 15,000 Daltons, and also includes multiple polyatomic metal atom complexes associated with each said carrier molecule, each of polyatomic metal atom complexes including multiple paramagnetic metal atoms which are magnetically coupled to collectively render each said polyatomic metal atom complex superparamagnetic;
applying to said animal a strong magnetic field which includes a gradient; and
applying to at least a portion of said animal an electromagnetic perturbation field at a radio frequency generally corresponding to a resonant frequency of a predetermined atomic nucleus at a magnetic field strength which falls within the range of field strengths applied to said animal by said strong magnetic field, and measuring the radio frequency emissions to define a spatial map of magnetic resonance characteristics within tissues of said animal.

18. A method for selective hysteresis heating, comprising the steps of:

identifying a living vertebrate animal to be heated;

introducing into the blood stream or body cavity of said animal an agent comprising a hydrophilic polymeric compound which comprises a carrier molecule with a molecular weight of greater than about 15,000 Daltons, and also includes multiple polyatomic metal atom complexes associated with each said carrier molecule, each of polyatomic metal atom complexes includng multiple paramagnetic metal atoms which are mutually ferromagnetically coupled to collectively render each said polyatomic metal atom complex superparamagnetic; and

applying radio frequency energy to said animal at a relatively high field intensity and energy.

19. A method of magnetic resonance imaging, characterised in that the body or material to be imaged contains an agent comprising a hydrophilic polymeric compound which comprises a carrier molecule with a molecular weight of greater than about 15,000 Daltons, and also includes multiple polyatomic metal atom complexes associated with each said carrier molecule, each of polyatomic metal atom complexes including multiple paramagnetic metal atoms which are magnetically coupled to collectively render each said polyatomic metal atom complex superparamagnetic.

20. A method according to claim 19, characterised by applying to said body or material a strong magnetic field which includes a gradient and applying to at least a portion of said body or material an eletromagnetic perturbation field at a radio frequency generally corresponding to a resonant frequency of a predetermined atomic nucleus at a magnetic field strength which falls within the range of field strengths applied by said strong magnetic field, and measuring the radio frequency emissions to define a spatial map of magnetic resonance characteristics.

21. A method of radio frequency heating, characterised in that the material or body to be heated contains an agent comprising a hydrophilic polymeric compound which comprises a carrier molecule with a molecular weight of greater than about 15,000 Daltons, and also includes multiple polyatomic metal atom complexes associated with each said carrier molecular, each of polyatomic metal atom complexes including multiple paramagnetic metal atoms which are mutually ferromagnetically coupled to collectively render each said polyatomic metal atom complex superparamagnetic.

22. The composition of any of claims 1 to 16 when prepared for use in or in connection with a surgical, therapeutical or diagnostic method on a living body.

Fig. 1

Figure 2

320

301    311    302    303    304

310    310    310

320

Fig. 3

EP 0 361 960 A2

Figure 4

Fig. 5A

Fig. 5B

Fig. 5C

Fig. 5D

Fig. 5E